# EUROPEAN PATENT APPLICATION

(11) **EP 3 081 158 A1**
(43) Date of publication of application: **19.10.2016**
(21) Application number: 16165591.5
(22) Date of filing: 15.04.2016
(51) Int. Cl.: A61B 5/024, A61B 5/046

(54) **BIOLOGICAL INFORMATION PROCESSING SYSTEM, BIOLOGICAL INFORMATION PROCESSING DEVICE, AND METHOD FOR GENERATING ANALYSIS RESULT INFORMATION**

(30) Priority: 17.04.2015 JP 2015084706; 17.04.2015 JP 2015084707
(71) Applicant: Seiko Epson Corporation, Tokyo 160-8801 (JP)
(72) Inventor: Koyama, Fumio, Nagano, 392-8502 (JP)
(74) Representative: Hoffmann Eitle

(57) **Abstract**

A biological information processing system includes processor including hardware. The processor performs: acquisition processing in which pulse wave information is acquired; processing in which analysis processing on the pulse wave information is performed to generate analysis result information; and output processing in which the analysis result information that is generated is outputted. The processor generates information for displaying distribution information of a pulse interval found on the basis of the pulse wave information and a reliability index of measurement of the pulse wave information, as the analysis result information.

## Description

### CROSS-REFERENCE

This application is based upon Japanese Patent Application No. 2015-084706 filed on April 17, 2015 and Japanese Patent Application No. 2015-084707 filed on April 17, 2015, the entire contents of which are incorporated herein by reference.

### BACKGROUND

### 1. Technical Field

The present invention relates to a biological information processing system, a biological information processing device, and a method for generating analysis result information, and the like.

### 2. Related Art

Arrhythmias are widely known as cardiovascular problems. For example, atrial fibrillation, which is an example of arrhythmias, needs to be treated in early stages because of the risk of blood clotting in the heart and consequent cerebral infarction. Traditionally, in a test to diagnose atrial fibrillation, it is common to have the patient wear a Holter electrocardiograph for 24 to 48 hours (usually 24 hours) and check the occurrence of atrial fibrillation from the electrocardiogram taken during that period.

However, since atrial fibrillation in its initial stage has a low frequency of occurrence, atrial fibrillation that occurs only occasionally can be overlooked in a Holter electrocardiographic test for a duration of 48 hours or less. Also, a patient with asymptomatic atrial fibrillation does not experience any symptoms and therefore, in many cases, does not visit a hospital and finds out that he/she has atrial fibrillation only after the condition becomes severe (for example, after cerebral infarction occurs). The reason for not being able to use a Holter electrocardiograph for a long period of time is that a plurality of electrodes needs to be attached to the chest of a human body and this raises the problem of skin rash caused by the electrodes themselves and tapes used to hold the electrodes. While electrocardiographs that can record data for 40 days have been developed recently, the problem of skin rash has not been solved and therefore it is not realistic to use even these electrocardiographs continuously for a long period of time.

In contrast, JP-A-2013-55982 discloses a technique in which a parameter equivalent to RR interval is found accurately from pulse wave information, thus finding atrial fibrillation on the basis of the pulse wave information. The pulse wave information can be acquired by a pulse wave sensor such as a photoelectric sensor, for example. A device having the pulse wave sensor can be implemented, for example, as a wristwatch-type wearable device. Therefore, it is possible to wear the device continuously for a long period of time, while restraining the possibility of skin rash or the like.

By using pulse wave information as in JP-A-2013-55982 or the like, it is possible to wear the device over a long period of time (for example, about three to ten days) and therefore it is possible to properly detect atrial fibrillation or the like in its initial stage.

### SUMMARY

An aspect of the invention relates to a biological information processing system including a processor including hardware. The processor performs: acquisition processing in which pulse wave information is acquired; processing in which analysis processing on the pulse wave information is performed to generate analysis result information; and output processing in which the analysis result information that is generated is outputted. The processor generates information for displaying distribution information of a pulse interval found on the basis of the pulse wave information and a reliability index of measurement of the pulse wave information, as the analysis result information.

Another aspect of the invention relates to a biological information processing system including a processor including hardware. The processor performs: acquisition processing in which pulse wave information is acquired; processing in which analysis processing on the pulse wave information is performed to generate analysis result information; and output processing in which the analysis result information that is generated is outputted. The processor generates first analysis result information for identifiably displaying a pulse wave abnormality period during a measurement period, and second analysis result information for displaying at least one of a pulse waveform and a pulse interval waveform during at least a section in the pulse wave abnormality period.

Still another aspect of the invention relates to a biological information processing device including a processor including hardware. The processor performs: acquisition processing in which pulse wave information is acquired; processing in which analysis processing on the pulse wave information is performed to generate analysis result information; and output processing in which the analysis result information that is generated is outputted. The processor generates information for displaying distribution information of a pulse interval found on the basis of the pulse wave information and a reliability index of measurement of the pulse wave information, as the analysis result information.

Yet another aspect of the invention relates to a biological information processing device including a processor including hardware. The processor performs: acquisition processing in which pulse wave information is acquired; processing in which analysis processing on the pulse wave information is performed to generate analysis result information; and output processing in which the analysis result information that is generated is outputted. The processor generates first analysis result information for identifiably displaying a pulse wave abnormality period during a measurement period, and second analysis result information for displaying at least one of a pulse waveform and a pulse interval waveform during at least a section in the pulse wave abnormality period.

Still yet another aspect of the invention relates to a method for generating analysis result information including: performing processing in which pulse wave information is acquired; and performing analysis processing on the pulse wave information and thus generating analysis result information for displaying distribution information of a pulse interval found on the basis of the pulse wave information and a reliability index of measurement of the pulse wave information.

Further another aspect of the invention relates to a method for generating analysis result information including: performing processing in which pulse wave information is acquired; and performing analysis processing on the pulse wave information and thus generating first analysis result information for identifiably displaying a pulse wave abnormality period during a measurement period, and second analysis result information for displaying at least one of a pulse waveform and a pulse interval waveform during at least a section in the pulse wave abnormality period.

### BRIEF DESCRIPTION OF THE DRAWINGS

The invention will be described with reference to the accompanying drawings, wherein like numbers reference like elements.
FIG. 1 shows an example of the configuration of a biological information processing system according to an embodiment.
FIG. 2 shows an example of information displayed by analysis result information.
FIG. 3 shows another example of information displayed by analysis result information.
FIGS. 4A and 4B show another example of information displayed by analysis result information.
FIGS. 5A and 5B show another example of information displayed by analysis result information.
FIGS. 6A and 6B show another example of information displayed by analysis result information.
FIG. 7 shows an example of information displayed by first analysis result information.
FIG. 8 shows an example of information displayed by second analysis result information.
FIG. 9 shows another example of information displayed by second analysis result information.
FIG. 10 shows another example of information displayed by second analysis result information.
FIG. 11 shows an example of analysis report displayed by analysis result information.
FIG. 12 shows an example of analysis report displayed by analysis result information.
FIG. 13 shows an example of displaying second analysis result information, using a new display window.
FIG. 14 shows an example of the detailed configuration of a biological information processing system according to an embodiment.
FIG. 15 shows an example of the configuration of an analysis processor.
FIGS. 16A and 16B are graphs showing result of frequency analysis processing based on electrocardiogram RR interval.
FIG. 17 is a flowchart explaining a flow of analysis processing according to an embodiment.
FIG. 18 is an explanatory view showing a technique for setting determination sections overlapping each other.
FIGS. 19A and 19B show an example of the configuration of a pulse wave measuring device.
FIG. 20 shows an example of the configuration of a pulse wave measuring device.
FIGS. 21A to 21C show an example of the configuration of a pulse wave measuring device and a biological information processing system.

### DESCRIPTION OF EXEMPLARY EMBODIMENTS

An embodiment relates to a biological information processing system including a processor including hardware. The processor performs: acquisition processing in which pulse wave information is acquired; processing in which analysis processing on the pulse wave information is performed to generate analysis result information; and output processing in which the analysis result information that is generated is outputted. The processor generates information for displaying distribution information of a pulse interval found on the basis of the pulse wave information and a reliability index of measurement of the pulse wave information, as the analysis result information.

In the embodiment, the processor generates information for displaying a reliability index as well as distribution information of a pulse interval, as analysis result information. Thus, it is possible to perform a display to allow the viewer to properly make a determination based on the distribution information of the pulse interval, or the like.

Also, in the embodiment, the processor may generate information for displaying the distribution information of the pulse interval during a measurement period and a change in the reliability index during the measurement period, as the analysis result information.

Thus, it is possible to display the distribution information of the pulse interval and the change in the reliability index (change in time series), targeting the measurement period.

Also, in the embodiment, the processor may generate information for displaying the distribution information of the pulse interval during the measurement period and a change in the reliability index during the measurement period, on a same screen, as the analysis result information.

Thus, since the distribution information of the pulse interval and the reliability index can be displayed on the same screen, it is possible to display the respective pieces of information in such a way that their relations can be easily visually recognized, or the like.

Also, in the embodiment, the reliability index may be information based on at least one of body motion information of a user and wearing state information about a measuring device mounted on the user's body.

Thus, it is possible to employ the information based on at least one of the body motion information and the wearing state information, as the reliability index.

Moreover, in the embodiment, the distribution of the pulse interval may be information in which a first axis is a time axis and in which a value based on the pulse interval is plotted on a second axis.

Thus, it is possible to use the fluctuation in time series in the value based on the pulse interval, as the distribution information of the pulse interval.

Also, in the embodiment, the distribution information of the pulse interval may be information in which the first axis is the time axis and in which difference information or proportion information of the pulse interval at a second timing in relation to the pulse interval at a first timing is plotted on the second axis.

Thus, it is possible to use the difference information or the proportion information of the pulse intervals at two different timings, as the value based on the pulse interval.

Also, in the embodiment, the processor may generate information in which the reliability index is displayed on the second axis, as the analysis result information.

Thus, since the distribution information of the pulse interval and the reliability index on the same axis, it is possible to display the respective pieces of information in such a way that their relations can be easily visually recognized, or the like.

Also, in the embodiment, the distribution information of the pulse interval may be information in which a dot with a value on a first axis being a value based on the pulse interval at a first timing and with a value on a second axis being a value based on the pulse interval at a second timing is plotted.

Thus, it is possible to employ the information in which the values based on the pulse intervals at two different timings are values on the respective axes forming a plane, as the distribution information of the pulse interval.

Also, in the embodiment, the processor may generate information in which the reliability index is displayed on a plane prescribed by the first axis and the second axis, as the analysis result information.

Thus, since the distribution information of the pulse interval and the reliability index can be displayed on the same plane, it is possible to display the respective pieces of information in such a way that their relations can be easily visually recognized, or the like.

Also, in the embodiment, the processor may generate first analysis result information for identifiably displaying a pulse wave abnormality period during a measurement period and second analysis result information for displaying at least one of a pulse waveform and a pulse interval waveform during at least a section in the pulse wave abnormality period. At least one of the first analysis result information and the second analysis result information may be information including the analysis result information.

Thus, since an outline of the timing of occurrence of the pulse wave abnormality period can be intelligibly displayed via the first analysis result information and detailed information about the pulse wave abnormality period can be displayed by the second analysis result information, it is possible to properly display information that is necessary for a doctor to make a diagnosis, or the like.

Also, in the embodiment, the processor may generate the first analysis result information for identifiably displaying the pulse wave abnormality period for determining an abnormal state of a cardiovascular system during the measurement period.

Thus, it is possible to generate the analysis result information for performing a display related to the abnormal state of the cardiovascular system.

Also, in the embodiment, the abnormal state of the cardiovascular system may be an arrhythmia.

Thus, it is possible to generate the analysis result information for performing a display related to the arrhythmia.

Another embodiment of the invention relates to a biological information processing system including a processor including hardware. The processor performs: acquisition processing in which pulse wave information is acquired; processing in which analysis processing on the pulse wave information is performed to generate analysis result information; and output processing in which the analysis result information that is generated is outputted. The processor generates first analysis result information for identifiably displaying a pulse wave abnormality period during a measurement period, and second analysis result information for displaying at least one of a pulse waveform and a pulse interval waveform during at least a section in the pulse wave abnormality period.

In this another embodiment of the invention, the processor generates the first analysis result information which makes the pulse wave abnormality period identifiable and the second analysis result information for displaying information during at least a section in the pulse wave abnormality period. Although a measurement period is expected to be relatively long in a technique using pulse wave information, it is possible, with the first analysis result information, to intelligibly display an outline of the timing of occurrence of the pulse wave abnormality period, or the like. Moreover, since detailed information about the pulse wave abnormality period can be displayed by the second analysis result information, it is also possible to properly display information that is necessary for a doctor to make a diagnosis, or the like.

Also, in this another embodiment of the invention, the processor may generate information for displaying a reliability index of measurement of the pulse wave information during the measurement period, as the first analysis result information.

Thus, it is possible to display the reliability index along with the pulse wave abnormality period.

Also, in this another embodiment of the invention, the reliability index may be information based on at least one of body motion information of a user and wearing state information about a measuring device mounted on the user's body.

Thus, it is possible to display the reliability index based on at least one of the body motion information and the wearing state information.

Also, in this another embodiment of the invention, if the reliability index in a first pulse wave abnormality period is higher than the reliability index in a second pulse wave abnormality period, the processor may generate the second analysis result information in such a way that analysis result information corresponding to the first pulse wave abnormality period is displayed preferentially over analysis result information corresponding to the second pulse wave abnormality period.

Thus, it is possible to change the degree of priority of display according to the reliability index in the case of displaying a plurality of analysis result information corresponding to a plurality of pulse wave abnormality periods by the second analysis result information, or the like.

Also, in this another embodiment of the invention, the processor may generate the second analysis result information in such a way that analysis result information corresponding to the first pulse wave abnormality period is displayed earlier than analysis result information corresponding to the second pulse wave abnormality period.

Thus, it is possible to preferentially display the analysis result information corresponding to the first pulse wave abnormality period, by making its display position (display order) precede.

Also, in this another embodiment of the invention, the processor may generate the second analysis result information in such a way that analysis result information corresponding to the second pulse wave abnormality period is displayed with a reduced size, compared with analysis result information corresponding to the first pulse wave abnormality period.

Thus, it is possible to preferentially display the analysis result information corresponding to the first pulse wave abnormality period, by changing the display size relatively.

Also, in this another embodiment of the invention, the processor may further perform section reliability determination processing in which the reliability index corresponding to each section in the measurement period is found on the basis of at least one of body motion information of a user and the pulse wave information.

Thus, it is possible to find the reliability index on the basis of at least one of the body motion information and the pulse wave information.

Also, in this another embodiment of the invention, the processor may further perform pulse interval calculation processing in which the pulse interval is found on the basis of the pulse wave information. The processor may perform determination processing on the pulse wave abnormality period on the basis of the pulse interval, and generate the first analysis result information on the basis of a result of the determination processing during each section in the measurement period and the reliability index corresponding to each section.

Thus, it is possible to perform the determination processing on the pulse wave abnormality period on the basis of the pulse interval, and generate the first analysis result information, using the determination result and the reliability index, or the like.

Also, in this another embodiment of the invention, the processor may generate information for displaying at least one of body motion information of a user during at least a section in the pulse wave abnormality period and wearing state information of the user, as the second analysis result information.

Thus, it is possible to display at least one of the body motion information and the wearing state information by the second analysis result information.

Also, in this another embodiment of the invention, the processor may generate information for displaying a display object that indicates the pulse wave abnormality period, as the first analysis result information.

Thus, it is possible to identifiably display the pulse wave abnormality period, by using the display object.

Also, in this another embodiment of the invention, the processor may generate information for displaying the pulse wave abnormality period and a period that is not the pulse wave abnormality period, in different image display forms from each other, as the first analysis result information.

Thus, it is possible to identifiably display the pulse wave abnormality period, by changing the image display form.

Also, in this another embodiment of the invention, the processor may generate the first analysis result information for identifiably displaying the pulse wave abnormality period for determine an abnormal state of a cardiovascular system during the measurement period.

Thus, it is possible to generate the analysis result information for performing a display related to the abnormal state of the cardiovascular system.

Also, in this another embodiment of the invention, the abnormal state of the cardiovascular system may be an arrhythmia.

Thus, it is possible to generate the analysis result information for performing a display related to the arrhythmia.

Also, in this another embodiment of the invention, the arrhythmia may be atrial fibrillation. The pulse wave abnormality period may be a period which is determined as having an occurrence of the arrhythmia by the analysis processing on the pulse wave information in the processor.

Thus, it is possible to generate the analysis result information for performing a display related to the atrial fibrillation.

Still another embodiment of the invention relates to a biological information processing device including a processor including hardware. The processor performs: acquisition processing in which pulse wave information is acquired; processing in which analysis processing on the pulse wave information is performed to generate analysis result information; and output processing in which the analysis result information that is generated is outputted. The processor generates information for displaying distribution information of pulse interval found on the basis of the pulse wave information and a reliability index of measurement of the pulse wave information, as the analysis result information.

Yet another embodiment of the invention relates to a biological information processing device including a processor including hardware. The processor performs: acquisition processing in which pulse wave information is acquired; processing in which analysis processing on the pulse wave information is performed to generate analysis result information; and output processing in which the analysis result information that is generated is outputted. The processor generates first analysis result information for identifiably displaying a pulse wave abnormality period during a measurement period, and second analysis result information for displaying at least one of a pulse waveform and a pulse interval waveform during at least a section in the pulse wave abnormality period.

Still yet another embodiment of the invention relates to a method for generating analysis result information, the method including: performing processing in which pulse wave information is acquired; and performing analysis processing on the pulse wave information and thus generating analysis result information for displaying distribution information of pulse interval found on the basis of the pulse wave information and a reliability index of measurement of the pulse wave information.

Further another embodiment of the invention relates to a method for generating analysis result information, the method including: performing processing in which pulse wave information is acquired; and performing analysis processing on the pulse wave information and thus generating first analysis result information for identifiably displaying a pulse wave abnormality period during a measurement period, and second analysis result information for displaying at least one of a pulse waveform and a pulse interval waveform during at least a section in the pulse wave abnormality period.

Hereinafter, an embodiment will be described. The embodiment below should not unduly limit the contents of the invention described in the appended claims. Not all of the configurations described in the embodiment are necessarily essential components of the invention.

### 1. Technique in This Embodiment

First, a technique in this embodiment will be described. As disclosed in JP-A-2013-55982, a technique in which a pulse wave signal (photoelectric pulse wave signal, pulse wave information) acquired by a photoelectric transducer (photoelectric sensor) worn by a test subject is analyzed so as to conduct a test to diagnose an arrhythmia such as atrial fibrillation is known. Here, the test to diagnose an arrhythmia refers to detecting a part (period, section) in which an arrhythmia is suspected, in order for a doctor to make a diagnosis.

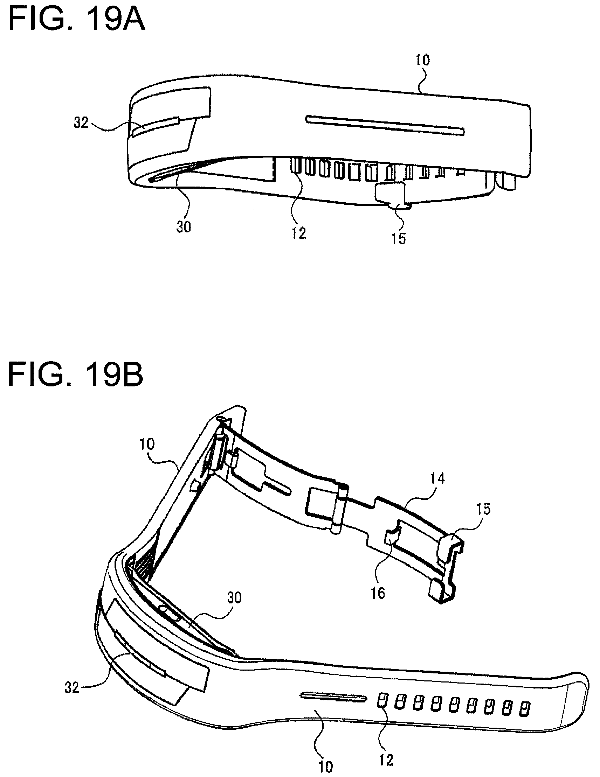

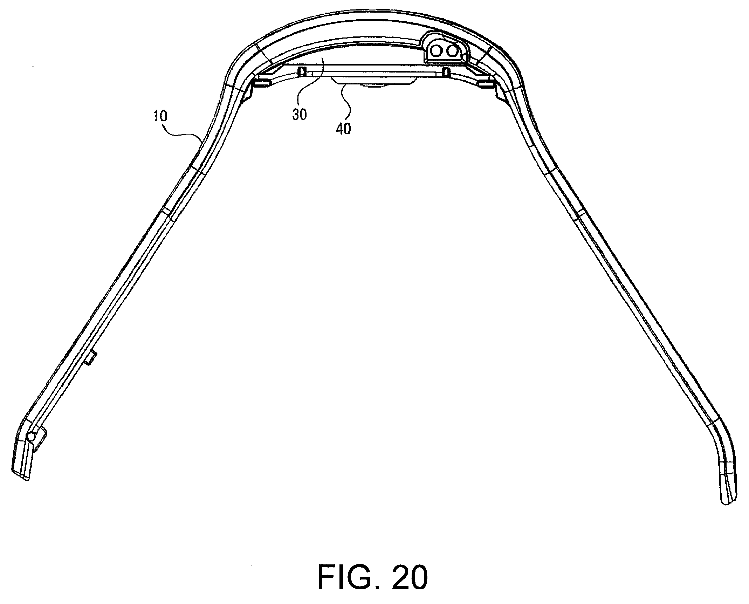

This test using a photoelectric pulse wave, compared with a test using a electrocardiograph, has an advantage that the device used is simpler and easier for the subject to wear, or that it suffices to press a device in the shape of a wristwatch or the like as shown in FIGS. 19A to 20 or the like in contact with the epidermis, causing less wearing load. This enables a longer-time test with an advantage that there is a high probability of catching a symptom onset phase that occurs irregularly. The site at which the pulse wave measuring device is fixed is not limited to the wrist and may be other sites such as the finger, neck, or ankle.

For example, atrial fibrillation is known as an arrhythmia. Atrial fibrillation is an abnormality in which a cardiac ventricle has convulsions (partial excitation and contraction), and the contraction of the cardiac ventricle occurs at irregular intervals. This causes the blood flow to stagnate. If atrial fibrillation continues for a long period of time, a thrombus can be formed easily. This can cause serious symptoms such as cerebral infarction and myocardial infarction.

Atrial fibrillation has a low frequency of occurrence in its initial stage. For example, it is possible that symptoms of atrial fibrillation appear only once in three days and for a few hours. In such a case, it is possible that the period of occurrence and the period when the Holter electrocardiograph is worn by the subject do not overlap with each other, resulting in an inability to detect atrial fibrillation. In contrast, a pulse wave measuring device (biological information processing device) for measuring pulse wave information can be easily worn for a longer period of approximately for three to ten days and therefore can detect atrial fibrillation even in the state where the frequency of occurrence is low.

Also, in the case of atrial fibrillation, asymptomatic atrial fibrillation of which the patient is unaware may be confirmed. In this case, it is unlikely that the patient will proactively have a test for atrial fibrillation. In some cases, there is a risk that symptoms may advance while the patient remains unaware of them, and may cause serious symptoms such as cerebral infarction. Even in this case, the use of pulse wave information is advantageous in that the patient can have a screening test with ease because the device can be easily worn for a long period.

However, the use of pulse wave information has a problem, too. Compared with measurement based on an electrocardiogram, measurement of pulse wave information is more affected by noises such as a body motion noise. Specifically, in the analysis of a photoelectric pulse wave signal, though the pulse wave analysis can be corrected by various kinds of noise elimination processing in the phase where the analysis is affected by a body motion noise, there can be no complete correction processing and the reliability of the result of the analysis of the pulse wave signal during that period drops. Also, in the phase of wearing failure, the amplitude of the pulse wave signal drops and the reliability equally drops.

Therefore, in the biological information processing system, even when distribution information of the pulse interval which is useful for determination on arrhythmias or the like is generated and displayed, the information can be wrong. For example, it is known that the variation in the pulse interval increases at the occurrence of an arrhythmia. However, even if the variation in the pulse interval is increased, it is necessary to carefully examine whether the increased variation is due to the user having an arrhythmia or due to the influence of a noise while the user is normal, and this causes a heavy burden.

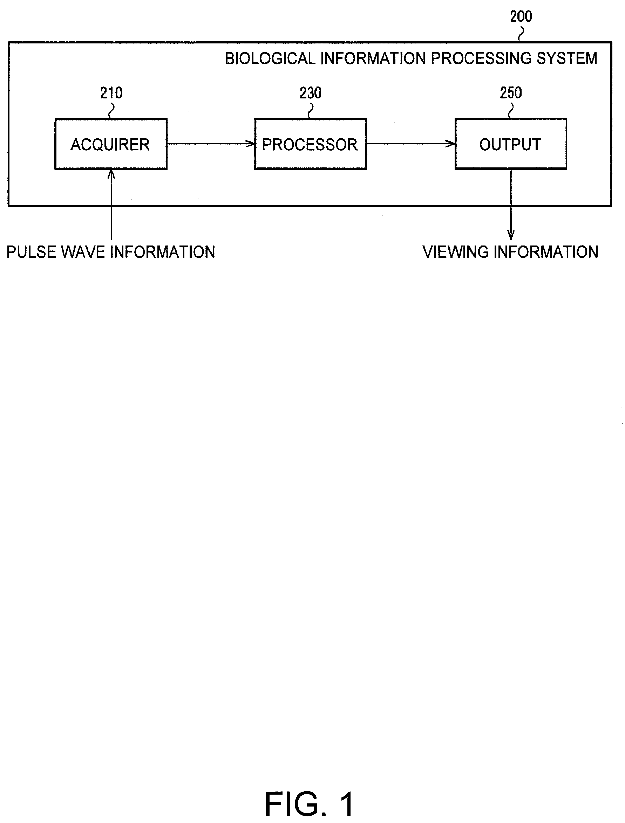

Thus, the present applicant proposes a technique in which a reliability index is displayed along with distribution information of a pulse interval. Specifically, a biological information processing system 200 according to the embodiment includes an acquirer 210 which acquired pulse wave information, a processor 230 which performs analysis processing of the pulse wave information and thus generates analysis result information, and an output 250 which outputs the generated analysis result information, as shown in FIG. 1. The processor 230 generates information for displaying distribution information of a pulse interval found on the basis of the pulse wave information and a reliability index of measurement of the pulse wave information, as the analysis result information.

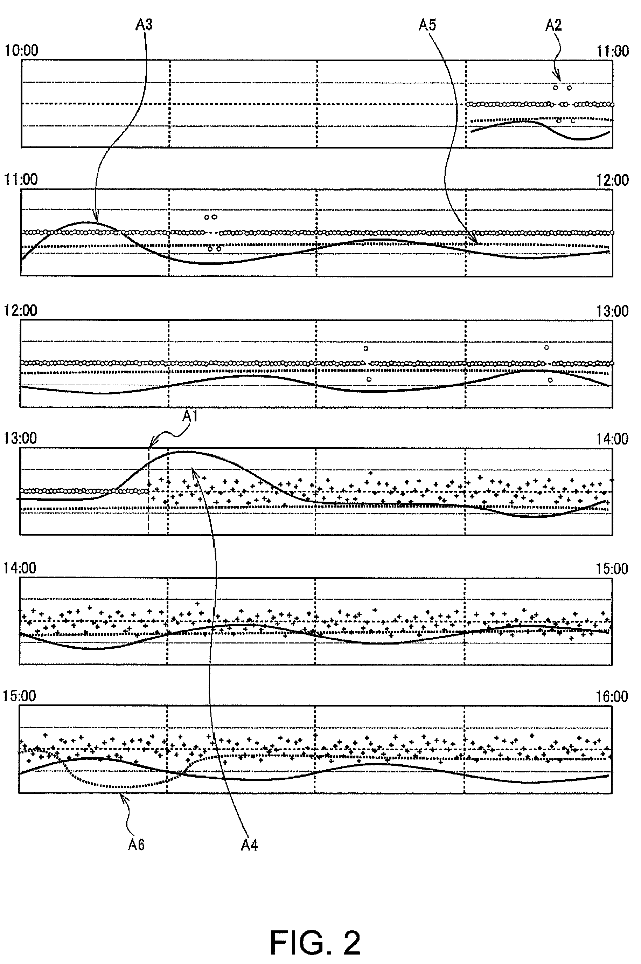

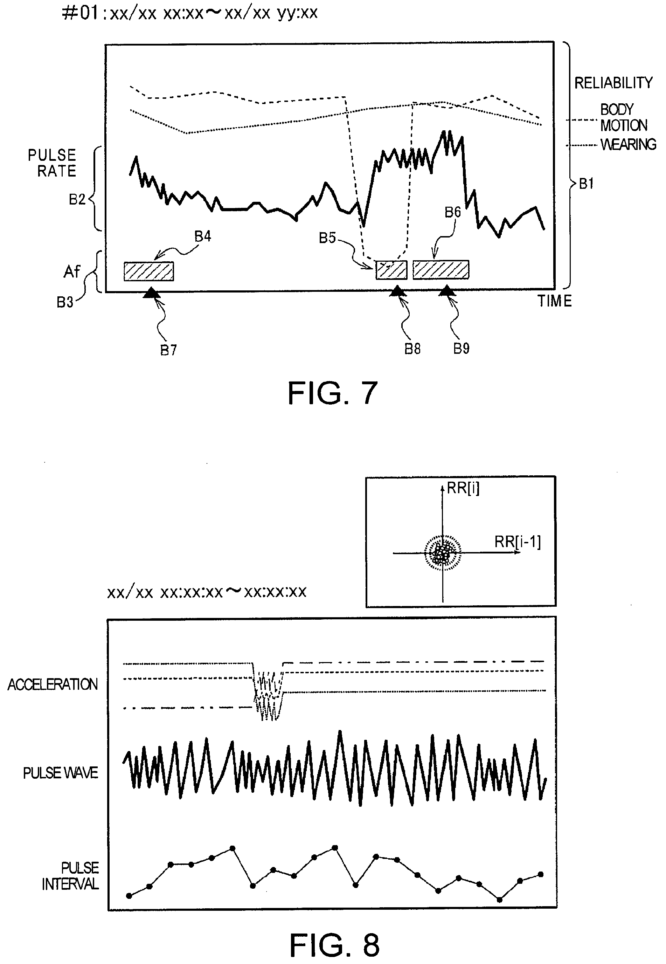

Here, the reliability index is information indicating to what extent the pulse wave measurement is reliable. An example of the reliability index is numerical value information and may be configured in such a way that a greater numerical value indicates that the pulse wave measurement is more reliable (higher reliability), whereas a smaller numerical value indicates that the pulse wave measurement is less reliability (lower reliability), or the other way round. Alternatively, a numerical value closer to a predetermined numerical value may indicate higher reliability. The relation between a specific value of the reliability index and high/low reliability can be implemented with various modifications. For example, in a curve A3 in FIG. 2, described later, since information based on the amount of effective signal of acceleration information is used as a reliability index, a greater numerical value indicates a larger body motion noise and lower reliability. Meanwhile, in FIG. 7, described later, a greater numerical value indicates higher reliability.

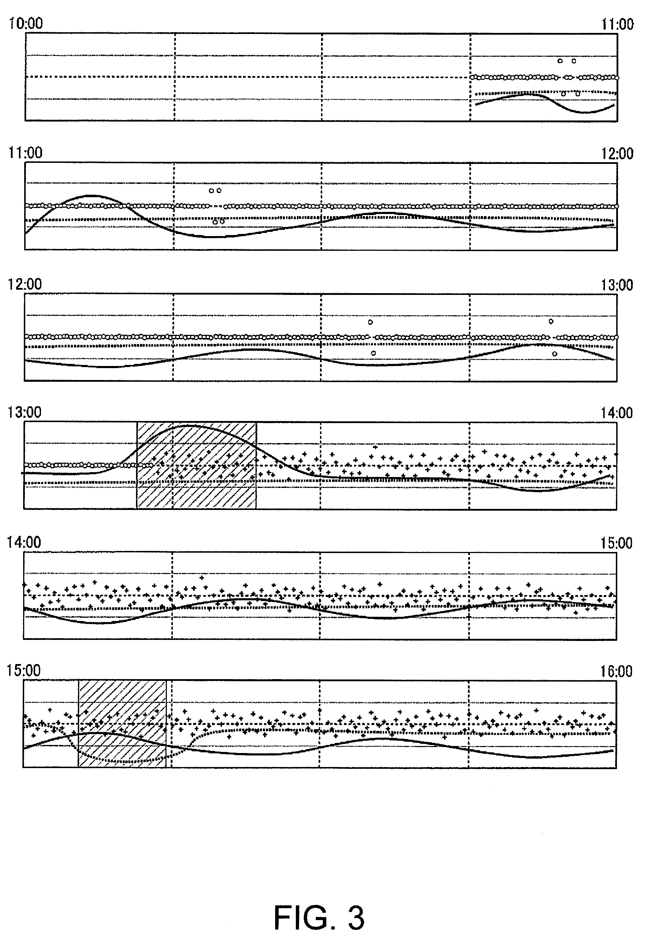

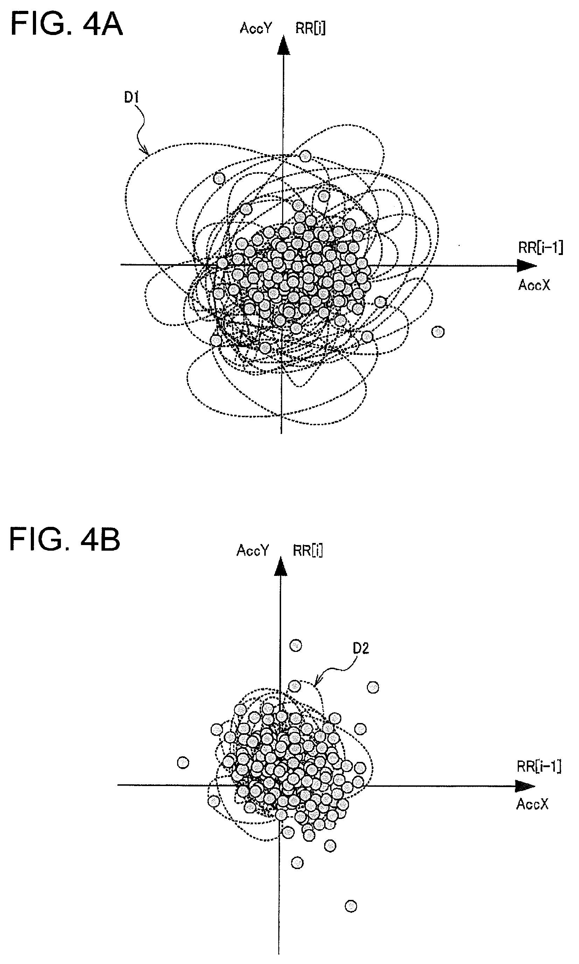

The pulse wave information is information indicating a pulse wave of a user (test subject, subject), and specifically, information based on an output from a pulse wave sensor (photoelectric sensor) worn by the user. The pulse interval is a period of time indicating the interval between one pulse to another (for example, in msec). For example, in relation to pulse rate (in rpm), which is broadly used, the pulse interval is in the relation of pulse interval = (1000x60) / pulse rate. The distribution information of the pulse interval is information indicating the value of the pulse interval or the variation in the value based on the pulse interval. Specifically, the distribution information may be information formed by plotting, in time series, the proportion of a pulse interval to the pulse interval at the immediately preceding timing, as described later with reference to FIGS. 2 and 3, or may be a plot known as a Lorenz plot or a plot similar to a Lorenz plot, as described later with reference to FIGS. 4A to 6B. The distribution information may also be other types of information. Details of the distribution information of the pulse interval will be described later.

Thus, since the distribution information of the pulse interval is displayed, it is possible to intelligibly present to the viewer (doctor) whether an arrhythmia is suspected or not. Also, since the reliability index is displayed as well, it is possible to intelligibly present whether the distribution of the pulse interval is reliable or not. Therefore, information that is useful for diagnosing an arrhythmia or the like can be properly presented. Specifically, in viewing the displayed information, the doctor only has to focus on information with large variation in the pulse interval and with high reliability. Even when pulse wave information over a long period of three to ten days is acquired, efficient viewing of information can be easily carried out.

If the biological information processing system 200 internally uses the reliability index and displays only the distribution information of the pulse interval corresponding to a section with high reliability, the amount of information viewed by the doctor can be reduced. However, an arrhythmia should be diagnosed by the doctor and it is not desirable to present only the result of determination by the biological information processing system 200. That is, since the decision on whether the outputted information is reliable or not needs determination by the doctor, it can be said that displaying the reliability index is very important.

Specifically, the processor 230 generates information for displaying the distribution information of the pulse interval during a measurement period and changes in the reliability index during the measurement period, as the analysis result information.

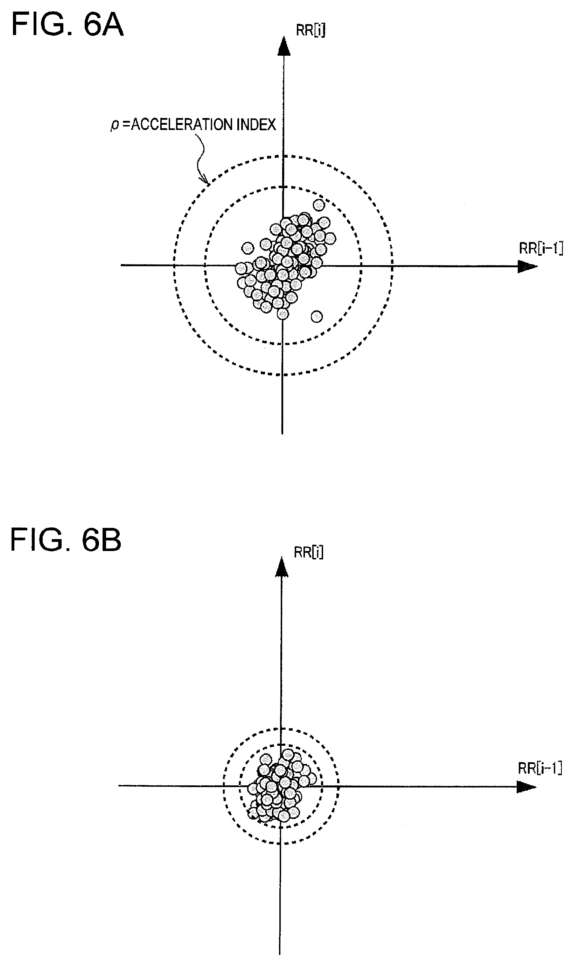

That is, targeting information during a predetermined period of time, the distribution information of the pulse interval and the reliability index may be displayed. The measurement period in this embodiment is a long period of three to ten days, as described above. However, the period (section) to be a target of display of the analysis result information may be a part of that period. In the example shown in FIGS. 2 and 3, described later, one axis is the time axis, and the distribution of the pulse interval during six hours and changes in time series in the reliability index are displayed. Meanwhile, in the examples of FIGS. 4A to 6B, a 20 to 60-second section is a target, and the distribution information of the pulse interval corresponding to a Lorenz plot and the reliability index (Lissajous figure of acceleration or the like) are displayed.

More specifically, the processor 230 may generate information for displaying the distribution information of the pulse interval during the measurement period and changes in the reliability index during the measurement period, on the same screen, as the analysis result information. In each of the examples shown in FIGS. 2 to 6B, the distribution information and the reliability index are displayed on the same screen. This enables the relation between the distribution information and the reliability index to be visually recognized at a time, and therefore enables an intelligible form of display. However, any display form of the analysis result information that enables recognition of the relation between the distribution information and the reliability index may be employed, and displaying the distribution information and the reliability index on separate screens is not precluded.

The arrhythmia in the embodiment is atrial fibrillation. The pulse wave abnormality period may be a period determined as having an occurrence of the arrhythmia, on the basis of the analysis processing on the pulse wave information by the processor 230. In an electrocardiogram, when the heart normally beats once, various characteristic waveforms such as the P-wave, Q-wave, R-wave, and S-wave appear. Therefore, various arrhythmias may be diagnosed by paying attention to differences between such normal waveforms and measured waveforms. Meanwhile, the pulse wave signal is for detecting fluctuations in the blood flow rate due to pulsation. Therefore, a pulse waveform does not have a shape as in the electrocardiogram. Thus, a technique using pulse wave information specializes in processing based on the pulse interval or pulse rate, rather than detecting various arrhythmias, and has a high affinity to detection processing of atrial fibrillation in which the variation in the pulse interval increases at the occurrence of the symptom.

Therefore, a case of atrial fibrillation will be described below, as an example. However, as an arrhythmia in the embodiment, employing arrhythmias other than atrial fibrillation (for example, premature contraction or the like) is not precluded.

Hereinafter, the analysis result information in the embodiment will be described in detail, including its modification examples, and then an example of the system configuration of the biological information processing system 200 or the like according to the embodiment will be described. Details of the processing by the processor 230 will be described along with the example of the system configuration.

### 2. Specific Example of Analysis Result Information

Details of the analysis result information in the embodiment will be described. Hereinafter, a display screen based on the analysis result information will be described as an example. However, the analysis result information may be screen information itself or may be other types of information that can generate screen information. Also, the screen described below is not limited to the display of an electronic apparatus or the like, and may be a printout on a paper medium or the like.

A specific example of the display screen where the distribution information of the pulse interval and the reliability index are displayed is shown in FIG. 2. In FIG. 2, the information is shown as divided in six rows. In each row, the horizontal axis represents time. For example, 10:00 in the top row indicates 10:00 (10 o'clock) on a predetermined day (or 10 hours 00 minutes past the start of the measurement period). That is, each row in FIG. 2 shows data corresponding to one hour, and information corresponding to a relatively long period of six hours can be displayed in the entirety of FIG. 2.

In FIG. 2, changes in time series in the pulse interval are plotted as dots. Specifically, the vertical axis in FIG. 2 may logarithmically express the proportion of the value of the pulse interval at a predetermined timing to the value of the pulse interval at the immediately preceding timing. That is, if a dot is plotted at the position of 0 on the vertical axis (in FIG. 2, the center position on the vertical axis), it means that the pulse interval at this timing is the same as the pulse interval at the previous timing. That is, in this graph, as dots are plotted at positions closer to 0, it means a smaller fluctuation in the pulse interval, whereas a greater variation in plotted positions indicates a greater fluctuation in the pulse interval.

Here, the term "plot" corresponds to processing in which, in the case where information prescribed by a set of a plurality of values and a space where one value corresponds to one dot (position) are provided, each value included in the set is arranged (displayed) in association with the space. In the example of FIG. 2, a set of proportion information of the pulse interval, which is one-dimensional values (scalar) acquired in time series, is arranged at corresponding positions in the direction of the vertical axis. Alternatively, it is also possible to consider that FIG. 2 is equivalent to processing in which a set of two-dimensional values (two-dimensional vectors) made up of proportion information and acquisition time is arranged in a two-dimensional space (plane) made up of a time axis and a proportion information axis. Also, the example of FIG. 4A or the like, described later, is equivalent to processing in which a set of two-dimensional values made up of the pulse interval at the immediately preceding timing and the pulse interval at the present timing acquired in time series is arranged in a two-dimensional space made up of two pulse interval axes.

In the example of FIG. 2, the distribution information of the pulse interval is information in which a first axis is the time axis and in which a value based on the pulse interval is plotted on a second axis. In the example of FIG. 2, the first axis is the horizontal axis and the second axis is the vertical axis. The value based on the pulse interval is a logarithm of the proportion to the pulse interval at the immediately preceding timing, as described above.

As the proportion to the immediately preceding timing is thus used, plotted dots concentrate around the position of a predetermined value (in this example, 0) if the variation is small, whereas plotted dots are dispersed in a broad range if the variation is large. That is, since changes in time series in the concentration and dispersion of plotted dots can be displayed, it is possible to make it easier for the viewer to diagnose the arrhythmia.

Here, the value of the pulse interval has the characteristic of fluctuating according to the state of the user (patient). For example, when the user is in a sleeping state or in a resting state, the pulse interval is long, whereas when the user is in an exercising state, the pulse interval is short. Even if the value of the pulse interval changes because of a shift from the sleeping state to the exercising state, the change is a normal reaction and not due to the arrhythmia. That is, the variation in the pulse interval can include variation due to a change in the state of the user (unrelated to the arrhythmia) and variation due to the arrhythmia. If the value of the pulse interval itself is plotted, the variation due to both factors is displayed. However, if the proportion to the immediately preceding timing is displayed, the influence of the variation due to the change in the state of the user, which is a longer-term change, can be restrained.

In view of this point, in the example of FIG. 2, the distribution information of the pulse interval is defined as information in which the first axis is the time axis and in which the proportion information of the pulse interval at a second timing to the pulse interval at a first timing is plotted on the second axis. However, the value on the second axis is not limited to the proportion information, since it suffices that the influence of the variation due to the change in the state of the user can be restrained. For example, the distribution information of the pulse interval may be information in which the first axis is the time axis and in which difference information of the pulse interval at a second timing from the pulse interval at a first timing is plotted on the second axis. The proportion information and the difference information may be the proportion itself or the difference itself, or may be information found from the proportion or the difference, or may be information corresponding to the proportion or the difference.

Each of the first timing and the second timing is an acquisition (calculation) timing of the pulse interval or a timing decided on the basis of the acquisition timing. As an example, a timing (for example, a timing indicating the end point), of a pulse interval calculation section, described later, may be employed. Since the proportion information and the difference information are used in view of intelligibly presenting the degree of fluctuation in the pulse interval as described above, the first timing and the second timing, in a narrow sense, are next to each other.

In the example of FIG. 2, before a timing indicated by A1, plotted dots are stable at positions substantially close to 0. Therefore, by viewing this information, the doctor can diagnose that the arrhythmia does not occur during this period. Although there are dots where there is a large fluctuation in the pulse interval from the immediately preceding timing, as indicated by A2 or the like, a variation to this extent can be seen in normal users and therefore poses no problem.

Meanwhile, after the timing indicated by A1, plotted dots are broadly dispersed and no regularity can be seen in the dispersion. Therefore, the doctor suspects the occurrence of the arrhythmia during this period.

Up to this point, the technique using the proportion information or the like, in order to restrain the influence of the fluctuation in the pulse interval unrelated to the arrhythmia, has been described. However, since the fluctuation in the pulse interval unrelated to the arrhythmia is similarly important information indicating the state of the pulse of the user, it is useful to present this information to the doctor. That is, a form of embodiment in which both the fluctuation in the pulse interval due to the change in the state of the user (unrelated to the arrhythmia) and the fluctuation in the pulse interval due to the arrhythmia are displayed is highly conceivable.

The fact that the influence of the fluctuation in the pulse interval unrelated to the arrhythmia can be restrained by using the proportion information or the difference information of the pulse interval means, in other words, that when the proportion information or the like is used, the fluctuation in the pulse interval due to the change in the state of the user is less likely to appear in the display. That is, if even the fluctuation in the pulse interval due to the change in the state of the user is to be displayed while using the proportion information or the like, it is desirable to separately display a change in time series in the pulse rate or the pulse interval.

Alternatively, the value of the pulse interval itself may be used as the value based on the pulse interval. As described above, the value of the pulse interval itself includes both the fluctuation due to the change in the state of the user and the variation due to the arrhythmia. Therefore, by using the value of the pulse interval, it is possible to express both at the same time. That is, the "value based on the pulse interval" used for displaying the distribution information of the pulse interval may be the value of the pulse interval itself or may be the proportion information or the difference information. Various modifications are possible.

By thus employing the display shown in FIG. 2, the viewer can presume whether atrial fibrillation has occurred or not, on the basis of the degree of dispersion of plotted dots. However, in the embodiment, the biological information processing system 200 may determine whether it is a pulse wave abnormality period or not, and the result of the determination may be displayed in an intelligible (highly visible) form.

Specifically, the processor 230 generates information for displaying a pulse wave abnormality period and a period that is not the pulse wave abnormality period in different image display forms, as the analysis result information. Various specific examples of the different image display forms are possible. For example, different dot shapes may be used, as shown in FIG. 2. In FIG. 2, cross-shaped dots are used for a pulse wave abnormality period, and solid white circular dots are used for a period that is not the pulse wave abnormality period. Thus, whether it is a pulse wave abnormality period or not can be easily discriminated on the basis of the dot shape, and the burden on the doctor can be reduced. In the example of FIG. 2, it can be readily understood that there is a possibility of atrial fibrillation occurring after the timing of A1 and at least until 16:00. Therefore, the doctor can make a decision to view detailed data corresponding to this period. Alternatively, modifications such as changing the color of dots between the pulse wave abnormality period and the other periods, changing the size, or changing the background color, are possible as well. Also, the image display form may be changed by flashing on and off the dots, background or the like in the case of displaying on the display of a predetermined device, though it is practically difficult in the case of printing an analysis report. The pulse wave abnormality period is a period in which it is detected that the pulse wave information is different from its normal state (in an abnormal state). An example of the pulse wave abnormality period is a period in which it is detected that the variation in the pulse interval is great, as described above. Details of the method for detecting the pulse wave abnormality period will be described later.

The processor 230 may generate first analysis result information for identifiably displaying a pulse wave abnormality period for determining an abnormal state of the cardiovascular system during the measurement period. That is, the pulse wave abnormality period may be information indicating whether it is a period in which an abnormality in the cardiovascular system is suspected or not. Also, the abnormal state of the cardiovascular system may be an arrhythmia.

The pulse wave sensor such as a photoelectric sensor can detect, for example, a fluctuation in the blood flow rate in a blood vessel. That is, an abnormality in the cardiovascular system, mainly in the blood vessel system, can be detected on the basis of the analysis processing on the pulse wave information. With the analysis result information according to the embodiment, the abnormal state of the cardiovascular system can be intelligibly presented to the viewer. Moreover, since the use of information such as the pulse rate or the pulse interval enables determination on whether the pulse of the user is in a normal state or not, it is possible to focus on an arrhythmia as the abnormal state of the cardiovascular system. In the description below, it is assumed that the pulse wave abnormality period is for determining whether there is an arrhythmia or not. Also, the arrhythmia in the embodiment, in a narrow sense, is atrial fibrillation, as described later.

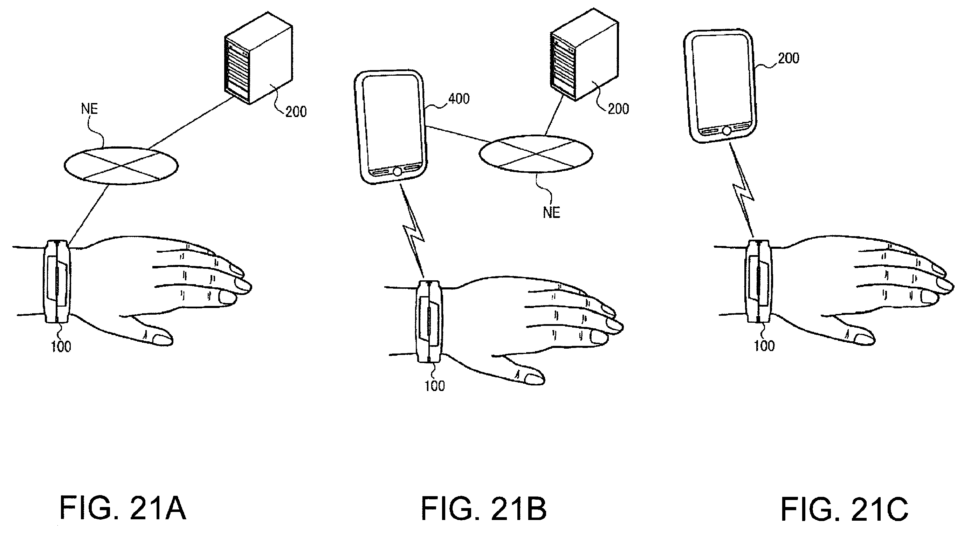

As shown in Fig. 2, the reliability index is displayed along with the display of information about the pulse interval. The reliability index in this case is information based on at least one of body motion information of the user and wearing state information of the user. Here, the information based on the body motion information may be the body motion information itself or may be information obtained as a result of performing certain processing on the body motion information. The information based on the wearing state information may be the wearing state information itself or may be information obtained as a result of performing certain processing on the wearing state information. The wearing state information may also be information indicating the wearing state of a measuring device mounted on the user' s body. The measuring device in this example is, for example, a pulse wave measuring device 100 as described later with reference to FIGS. 19A to 20. As described later with reference to FIGS. 21A to 21C, the biological information processing system 200 according to the embodiment may be implemented by a device such as a server system or may be implemented by the pulse wave measuring device 100. That is, the measuring device in this example may refer to a device which is different from a device including the biological information processing system 200, or may be refer to a device including the biological information processing system 200.

A3 in FIG. 2 indicates a curve showing changes in time series in the reliability index related to body motion noise. The vertical axis represents the magnitude of the body motion noise. Here, the body motion noise is higher as it goes upward on the vertical axis, and lower as it goes downward on the vertical axis. Specifically, an amount of effective signal of the body motion information or the like may be used as the reliability index.

Thus, it is possible to intelligibly present that if the value on the curve A3 is large (situated in an upper part on the vertical axis), the body motion noise is high and the reliability of measurement of pulse wave information is low. Therefore, while the biological information processing system 200 determines that the period indicated by A4 is a pulse wave abnormality period, the doctor can be allowed to make a decision that the possibility that the variation in the pulse interval during this period may be due to the body motion noise cannot be eliminated. Displaying the reliability index enables the doctor to easily determine whether large variation in the distribution information of the pulse interval is due to an arrhythmia or due to a noise, when it happens. Also, it is possible to differentiate pulse wave abnormality periods according to the reliability, instead of treating all pulse wave abnormality periods equally, and to reduce the burden on the doctor further.

A5 in FIG. 2 indicates information based on the wearing state information. Specifically, the vertical axis may represent the effective value of the pulse wave signal (effective value of the amplitude of the pulse waveform). Thus, it is possible to intelligibly present that if the value on the curve A5 is small (situated in a lower part of the vertical axis), the signal level is low, the wearing state is not good, and the reliability of measurement of the pulse wave information is low. Specifically, it is possible to easily present to the doctor that the reliability during a period A6 is low, as in the period A4.

For a period which is determined as a pulse wave abnormality period with low reliability, such as A4 and A6, further highlighting may be carried out to discriminate this period from periods with high reliability. As an example, the background color in the section with lower reliability may be changed, as shown in FIG. 3. Thus, it is visually clarified that the determination based on the pulse wave information is wrong with respect to the section where the background color is changed. Therefore, the doctor only has to make a diagnosis, focusing on the section where the background color is not changed (or the section where the background color indicates high reliability). Thus, the burden of viewing information can be reduced.

In the above example, the processor 230 generates information in which the reliability index is displayed on the second axis, as the analysis result information. By thus combining the axis on which the distribution information of the pulse interval is displayed and the axis on which the reliability index is displayed, it is possible to display the distribution information and the reliability index on the same screen (in a narrow sense, superimpose the distribution information and the reliability index) and therefore to intelligibly present the relation between these. Here, the axis on which the reliability index is displayed only needs to be in the same direction as the axis on which the distribution information is displayed, and need not be the same axis. Specifically, the processor 230 may generate information in which the reliability index is displayed on a third axis in a direction corresponding to the second axis, as the analysis result information.

In FIGS. 2 and 3, an example of screen where the distribution information of the pulse interval and the reliability index over a relatively long period are displayed is explained. However, the analysis result information in the embodiment is not limited to this example and may be information in which information over a shorter period is displayed.

A specific example is shown in FIGS. 4A and 4B. In FIGS. 4A and 4B, the distribution information of the pulse interval is information in which dots with a value on a first axis being a value based on the pulse interval at a first timing and with a value on a second axis being a value based on the pulse interval at a second timing are plotted.

If, with respect to predetermined information that changes in time series, the value at a predetermined timing is f[i] and the value at the immediately preceding timing is f[i-1], a group of dots with (x, y) = (f[i], f[i-1]) plotted on an xy plane at various timings i is known as a Lorenz plot. In the Lorenz plot, if the value does not change from the immediately preceding timing, dots are plotted on a straight line y=x, and as the change in the value becomes greater compared with the immediately preceding timing, dots are plotted at positions more away from the straight line y=x. That is, with the Lorenz plot, it is possible to determine the degree of variation in value by looking at whether the plotted dots concentrate near y=x or are dispersed in a broad range.

That is, in the case where the Lorenz plot is prepared using the pulse interval, if dots concentrate near the straight line y=x, the pulse interval varies less. That is, it can be determined that there is no occurrence of an arrhythmia. Meanwhile, if dots are dispersed to positions away from the straight line y=x, it can be determined that the pulse interval varies, that is, the occurrence of an arrhythmia is suspected.

In the example of FIGS. 4A and 4B, the average value of pulse intervals in a 20 to 60-second section that is to be a display target is found, and a value on the first axis is a differential value between the pulse interval at a first timing and the average value, whereas a value on the second axis is a differential value between the pulse interval at a second timing and the average value. Since the pulse interval only has a positive value, if a normal Lorenz plot is prepared using the pulse interval itself, dots are distributed in the first quadrant. However, if differential values from the average value are employed in this manner, dots are plotted about the origin as the center and the degree of dispersion of the plotted dots can be intuitively understood more easily.

Also, since the origin is defined as the center, the relation with the reliability index can be intelligibly displayed as well. For example, the processor 230 generates information in which the reliability index is displayed on the plane prescribed by the first axis and the second axis, as the analysis result information.

In the example of FIG. 4A, a value on a first axis of acceleration information at a predetermined timing t (for example, a value AccX[t] on the x-axis in an xy two-axis acceleration sensor or an xyz three-axis acceleration sensor) may be plotted on the first axis, and a value on a second axis of the acceleration information (for example, an AccY[t] on the y-axis) may be plotted on the second axis, and the plotted dots may be connected by a smooth curve to draw a figure. For example, if acceleration information is acquired at 16 Hz and FIG. 4A is focused on a 20-second section, 480 dots are plotted with respect to the acceleration information. Therefore, these dots may be connected to draw a figure. This corresponds to a curve D1 in FIG. 4A. Here, AccX and AccY may be information obtained by extracting a specific band with a digital filter. The specific band is, for example, 0.5 to 2.0 Hz, which overlaps with the frequency of pulse.

In this case, as the value of acceleration becomes greater, the drawn curve passes through positions more away from the origin. That is, if the acceleration is high and the influence of the body motion noise is large, the curve passes through many positions more away from the origin, as indicated by D1 in FIG. 4A, and therefore results in a shape spreading largely from the origin. Meanwhile, if the acceleration is low and the influence of the body motion noise is small, the curve passes through many positions closer to the origin, as indicated by D2 in FIG. 4B, and therefore remains around the center with little spread from the origin. That is, D1 in FIG. 4A and D2 in FIG. 4B are proper indications as the reliability index of the body motion noise.

In the example of FIGS. 4A and 4B, an arrhythmia is suspected if the distribution information of the pulse interval spreads largely, and lower reliability (higher body motion noise) is suspected as the reliability index spreads more largely. That is, the viewer only had to view the spreads of these two types of information from the origin and therefore can easily understand what the information means.

For example, both FIGS. 4A and 4B are circumstances where the distribution information of the pulse interval spreads largely and therefore an arrhythmia is suspected, as can been seen in comparison with FIGS. 6A and 6B, described later. However, in FIG. 4A, since the reliability index spreads largely, too, the possibility that the spread of the distribution information of the pulse interval may be due to the body motion noise, that is, that no arrhythmia may have occurred, can be considered. Meanwhile, in FIG. 4B, since the spread of the reliability index is small, it can be determined that the spread of the distribution information of the pulse interval is due to an arrhythmia. That is, by superimposing the reliability index, it is possible to properly determine the factor that causes the spread of the distribution information of the pulse interval and thus reduce the burden on the doctor.

As the reliability index in this example, changes in value need not be displayed in time series, since it suffices that the magnitude of the body motion noise, more specifically, the magnitude of the acceleration information, can be seen. For example, the amount of effective signal, which is an index value of the magnitude of the acceleration information, may be found and this amount of effective signal may be displayed on the plane prescribed by the first axis and the second axis.

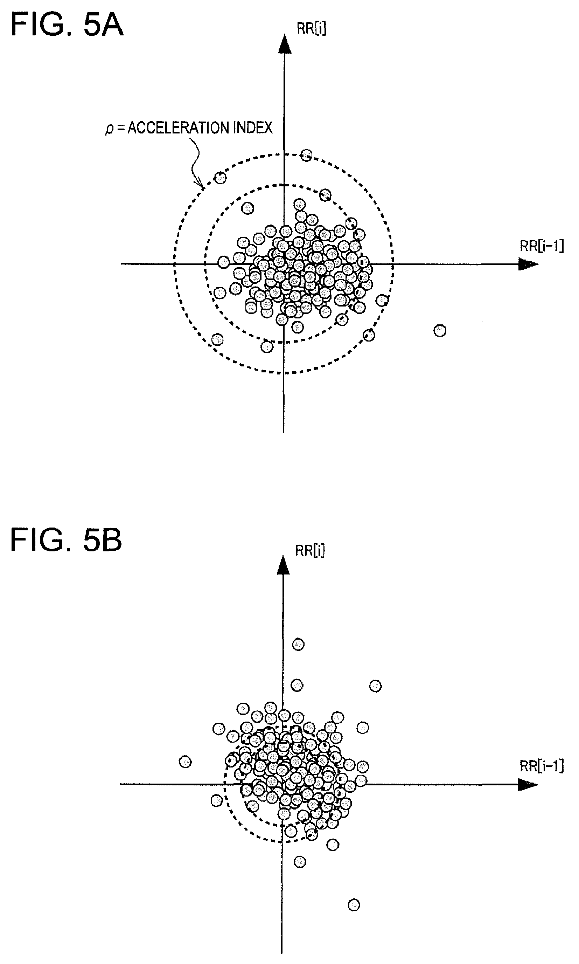

An example of this is FIGS. 5A and 5B. In FIGS. 5A and 5B, an amount of effective signal p of the acceleration information is found and a circle with a radius a p is superimposed in the display. Specifically, the power in a predetermined band (for example, 0. 5 to 2 Hz as described above) of accelerations on three axes may be used. Also, information on each axis may be converted by being multiplied by a coefficient corresponding to the degree of influence of each axis on the pulse wave measurement, instead of treating information on the three axes equally. Various modifications are possible.

Since p increases as the value of acceleration becomes greater, if the circle has a large radius as in FIG. 5A, it can be determined that the acceleration is high, that is, the body motion noise is high and the reliability is low. Meanwhile, if the circle has a small radius as in FIG. 5B, it can be determined that the acceleration is low, that is, the body motion noise is low and the reliability is high. That is, also in the display as shown in FIGS. 5A and 5B, it is possible to intelligibly show the relation between the distribution information of the pulse interval and the reliability index in a superimposed manner.

FIGS. 4A to 5B are all examples of the state where an arrhythmia (atrial fibrillation) has occurred. In contrast, an example of screen display of analysis result information in the state where there is no occurrence of atrial fibrillation is shown FIGS. 6A and 6B. FIG. 6B shows the state where the body motion noise is low and there is no occurrence of an arrhythmia. As clear from the illustration, the distribution information of the pulse interval concentrates near the origin. FIG. 6A shows the state where there is no occurrence of an arrhythmia but the body motion noise is high. As can be seen from the illustration, in FIG. 6A, the distribution information of the pulse interval spreads more broadly than in FIG. 6B, but the circle has a greater radius p. Therefore, it can be presumed that the spread is due to the influence of the noise and that atrial fibrillation has not occurred.

As the case where the distribution information of the pulse interval spreads largely, three specific circumstances are conceivable. That is, a first case where the body motion noise is low and atrial fibrillation has occurred, as shown in FIG. 5B, a second case where the body motion noise is high and atrial fibrillation has occurred, as shown in FIG. 5A, and a third case where the body motion noise is high and atrial fibrillation has not occurred, are conceivable.

In the first case, since the reliability is high, it can be understood that there is a high possibility that atrial fibrillation has occurred. Therefore, this case poses no particular problem. Meanwhile, with respect to how to handle the second and third cases, several methods are conceivable. As an embodiment of the invention, it is possible not to discriminate the second case and the third case, and not to attach importance to any of the information in these cases in diagnosing an arrhythmia because the body motion noise is high and the reliability is low. That is, the idea is that, though atrial fibrillation may or may not have occurred in practice, the information is not actively used since the body motion noise is high and the reliability is low.

However, in the embodiment, the second case and the third case are discriminated from each other. That is, a display that enables determination on whether atrial fibrillation has occurred or not, even if the body motion noise is high, may be provided. Specifically, in each of the above examples, by properly adjusting the scale of two elements, it is possible to easily determine whether the variation in the pulse interval is genuinely biologically caused or is possibly affected by the body motion noise.

More specifically, if the distribution information of the pulse interval varies beyond the spread of the Lissajous figure or circle, as shown in FIGS. 4A and 5A, it can be said that there is a high possibility that an episode of atrial fibrillation (occurrence of atrial fibrillation) may be suspected. Meanwhile, if the distribution information of the pulse interval varies but the Lissajous figure or the circular indicator spreads beyond this, as shown in FIG. 6A, it can be said that the possibility of false positive due to the influence of the body motion noise cannot be eliminated. That is, by properly setting the scale, when the distribution information of the pulse interval spreads largely, it is possible to determine whether the large spread is biologically caused or due to the body motion noise.

In the examples of FIGS. 5A to 6B, if the amount of effective signal of acceleration information is p, the scale on each axis may be set in such a way that the range to which the distribution information of the pulse interval can spread due to this acceleration (body motion noise) and the value of the amount of effective signal p are equivalent to each other. For example, a healthy user without atrial fibrillation is made to do a certain kind of exercise, and pulse wave information and acceleration information during the exercise are measured. Then, the amount of effective signal p and the degree of spread of the distribution information of the pulse interval (as an example, the distance to a dot plotted at the farthest position from the origin) are calculated. If this process is carried out with respect to a plurality of users and a plurality of kinds of exercise, multiple data indicating the relation between the amount of effective signal p and the spread of the distribution information of the pulse interval can be acquired. Using a statistical method on such data, for example, the relation that when the amount of effective signal is p, the spread of the distribution information of the pulse interval is expected to fall within this range, can be derived. Therefore, the scale on each axis may be set on the basis of this relation.

With such scale setting, if the distribution information of the pulse interval varies beyond the circle, as shown in FIG. 5A, it can be determined that the variation is too large to be explained by the body motion noise alone, that is, there is a high possibility that atrial fibrillation has occurred. Meanwhile, if the distribution information of the pulse interval concentrates inside the circle, as shown in FIG. 6A, it is understood that the variation in the distribution information is due to the body motion noise and therefore it can be determined that there is a high possibility that atrial fibrillation is absent.

The display screen showing the analysis result information in the embodiment is not limited to those shown in FIGS. 4A to 6B. Various modifications can be made such as using a circular indicator on a third axis in addition to the Lissajous trajectory taken on the two axes shown in FIGS. 4A and 4B, or displaying wearing state information as the circular indicator, for example. In the case of displaying wearing state information as the circular indicator, the radius of the circle needs to be increased as the wearing state becomes worse, in order to achieve a display similar to the above. That is, when the amount of effective signal of the pulse wave information is used as the wearing state information, a value based on the reciprocal of the amount of effective signal may be used as the radius of the circle.

### 3. Modifications

In the case of the method using the pulse wave information, other problems that are not anticipated in the case of using an electrocardiogram can occur. Specifically, useful information for swift diagnosis needs to be reported efficiently from long-time data. As described above, the pulse wave information is acquired over a period of about three to ten days and therefore results in a massive amount. Therefore, if detailed information, for example, a pulse waveform, which is the waveform of the pulse wave information, is displayed over the entire test period, the doctor has to bear a heavy burden of viewing the information. Therefore, it is preferable that analysis processing on the pulse wave information (for example, determination processing to determine whether an abnormality is seen in the pulse wave information or not) is performed on the side of the biological information processing system and that outline information using the result of the determination is presented.

However, an arrhythmia should be diagnosed by the doctor and it is not desirable to present only the result of the determination by the biological information processing system 200. In other words, the output (analysis report or the like) from the biological information processing system 200 must also present information that is detailed enough for the doctor to diagnose whether there is an arrhythmia or not. Particularly, the pulse wave information is influenced by the body motion noise or the like and has lower accuracy than in the case of using an electrocardiogram. Therefore, there is a great need for a screen display or the like for checking whether the result of detection of a pulse wave abnormality by the system is appropriate or not.

In short, the biological information processing system needs to generate and output information that satisfies both a first demand that the doctor should avoid viewing all of a massive amount of data and a second demand that information that is detailed enough for the doctor to be able to diagnose an arrhythmia is made available for viewing.

Thus, the processor 230 may generate first analysis result information for identifiably displaying a pulse wave abnormality period for determining an arrhythmia during the measurement period, and second analysis result information for displaying at least one of a pulse waveform and a pulse interval waveform during at least a section in the pulse wave abnormality period. Here, one of the first analysis result information and the second analysis result information is information including the analysis result information described above.

The pulse waveform is the waveform of the pulse wave information, and for example, a waveform showing changes in time series in the pulse wave information. Specifically, the pulse waveform may be a waveform indicating an AC component of a pulse wave sensor output. Also, the pulse interval waveform is a waveform showing changes in time series in the pulse interval.

With this configuration, it is possible to identify the pulse wave abnormality period during the measurement period by using the first analysis result information, and therefore it is possible to allow the doctor to easy grasp the outline of the situation of the arrhythmia during the measurement period. That is, even if the measurement period is a long period of three to ten days, at which timing and at what frequency the arrhythmia is suspected can be easily grasped. Therefore, the burden on the doctor can be reduced.

Moreover, by using the second analysis result information, it is possible to display detailed information such as the pulse waveform or the pulse interval waveform, targeting at least a section in the pulse wave abnormality period. Therefore, by using this detailed information, it is possible to allow the doctor to diagnose whether an arrhythmia truly occurs during the pulse wave abnormality period.

For example, the processor 230 may generate information for displaying the reliability index of measurement of the pulse wave information during the measurement period, as the first analysis result information. Since the pulse wave information used in the embodiment can be affected by noises relatively easily, such a reliability index is an important element. In other words, the concept of reliability index is not seen in the related-art technique using an electrocardiogram with relatively high accuracy.

Thus, if a plurality of pulse wave abnormality periods is displayed via the first analysis result information, a period with higher reliability, that is, a period during which not a noise but the occurrence of an abnormality in the user is suspected can be used preferentially for diagnosis, and the burden at the time of diagnosis can be reduced.

Hereafter, a specific example of the first analysis result information and a specific example of the second analysis result information (one episode waveform) will be described, and then an example of display in the case where a plurality of episode waveforms is included in the second analysis result information will be described, taking analysis report as an example. The analysis report in this example is information that is expected to be generated and outputted once corresponding to one round of test (measurement period), and is output information of the biological information processing system 200 including both the first analysis result information and the second analysis result information.

In the following description, it is assumed that, prior to the preparation of the analysis result information, the pulse wave information and the body motion information are acquired and that, on the basis of these, the pulse interval is found at a first rate (for example, once every second), and the result of determination on whether it is a pulse wave abnormality period or not, and the reliability index of pulse wave measurement are found at a second rate (for example, once every 20 seconds, or it may be the same as the first rate in some cases). Details of processing to find each type of information will be described later, along with an example of system configuration.

### 3.1 First Analysis Result Information

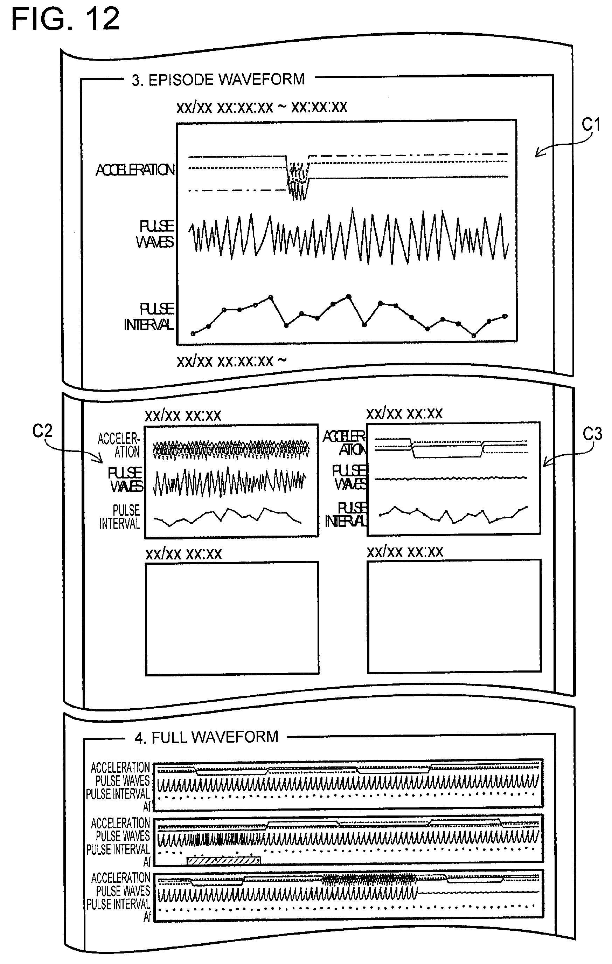

For example, if the pulse waveform or the pulse interval waveform is displayed over the entire measurement period, as shown in the full waveform of FIG. 12, described later, it is possible to display, without exception, information corresponding to the state where atrial fibrillation is occurring. However, FIG. 12 shows only a part of the full waveform, and viewing all the data taken over three to ten days puts a heavy burden on the doctor. Even if the doctor tries to extract and focus on a part of the information, with only the full waveform displayed, there is few clues to which part should be extracted and the doctor may end up having to check all the data.

That is, the analysis result information in the embodiment needs to show data over a relatively long period (in a narrow sense, the entire measurement period) in a form that enables understanding at a glance and to intelligibly present to the doctor a part to be a high-priority target of viewing. Therefore, in the embodiment, the first analysis result information for identifiably displaying a pulse wave abnormality period in the measurement period is generated, as described above.

As the first analysis result information, the analysis result information described above with reference to FIGS. 2 and 3 may be used. As described above, in FIG. 2, when displaying the distribution information of the pulse interval, cross-shaped dots are used for a pulse wave abnormality period, and white solid circular dots are used for periods other than the pulse wave abnormality period. Thus, whether it is a pulse wave abnormality period or not can be easily discriminated on the basis of the shape of the dots, and the burden on the doctor can be reduced.

Since it suffices that the first analysis result information in the embodiment enables identification of whether it is a pulse wave abnormality period or not, its display content is not limited to FIG. 2 or 3. For example, the processor 230 may generate information for displaying a display object (icon, mark) indicating the pulse wave abnormality period, as the first analysis result information.

A specific example of screen is shown in FIG. 7. In FIG. 7, the horizontal axis represents time. In an area B1, changes in time series in the reliability index are displayed. In an area B2, changes in time series in the pulse rate are displayed. Also, in an area B3, a display object (in the example of FIG. 7, a bar) is displayed in a range corresponding to a period determined as a pulse wave abnormality period.

In the example of FIG. 7, it is shown that three pulse wave abnormality periods B4 to B6 indicated by bars are detected in a display target period (for example, a predetermined day of the measurement period). The viewer can easily understand in which period the pulse wave abnormality periods are detected, on the basis of the presence/absence of bars and the positions of the bars on the horizontal axis.

In the example of FIG. 7, when displaying the reliability index, it is assumed that the reliability is higher as it goes upward on the vertical axis, and lower as it goes downward. That is, in the case of the reliability index based on the body motion noise, the reliability index is situated downward as the amount of body motion noise (for example, the amount of effective signal of acceleration information) becomes greater. In this respect, the example of FIG. 7 is different from the display of the reliability index in FIG. 2. In this way, the method for displaying the reliability index can be implemented with various modifications.

Since the occurrence of atrial fibrillation may continue for several tens of minutes in some cases, one pulse wave abnormality period may have a length of several tens of minutes as well. As described later, the second analysis result information displays information corresponding to at least a section (for example, a predetermined 20-second or 1-minute section) in a pulse wave abnormality period. Therefore, in the case of displaying the information of one pulse wave abnormality period with the use of the second analysis result information, information immediately after the start of this pulse wave abnormality period may be displayed, or information immediately before the end of the pulse wave abnormality period may be displayed. Also, information corresponding to an intermediate timing may be displayed.

Thus, since a section displayed using the second analysis result information can be freely set, which section in the pulse wave abnormality period is used for the display via the second analysis result information may be shown to the viewer. This enables the doctor to understand which section in the pulse wave abnormality period the data currently viewed corresponds to, when viewing the second analysis result information. For example, triangular objects as indicated by B7 to B9 in FIG. 7 may be shown. In the case of FIG. 7, it is shown that information during the sections corresponding to the vertices of the triangular objects, of the pulse wave abnormality periods, is displayed via the second analysis result information.

As described above, the processor 230 may perform determination processing on the pulse wave abnormality period on the basis of the pulse interval and generate the first analysis result information on the basis of the result of the determination processing with respect to each section in the measurement period and the reliability index for each section. Thus, the result of the determination processing (in FIGS. 2 and 3, the shape of plotted dots, and in FIG. 7, the presence/absence of a bar), and the reliability index (in FIGS. 2 and 3, A3 and A5, and in FIG. 7, the area B1) can be displayed together and therefore it is possible to show a display that reduces the burden of viewing on the doctor.

### 3.2 Second Analysis Result Information

Next, the second analysis result information will be described. The first analysis result information displays information over a long period of six hours in FIG. 2 and 24 hours in FIG. 7. Therefore, this information is not suitable for the display of information with small changes in time series, such as the pulse wave information acquired at a rate of 16 Hz and the pulse interval (pulse rate) acquired once every second. For example, the display of the pulse interval in FIG. 2 is for the purpose of grasping the trend of the distribution (dispersion) of dots, rather than paying attention to each dot. In the display of the pulse rate in FIG. 7, the information has to be thinned to a certain extent. Also, in FIGS. 2 and 7, it is difficult to display a pulse waveform acquired at a higher rate.

That is, only with the display of the first analysis result information, the displayed information is insufficient with some information missing, compared with the pulse wave information and the body motion information acquired by the acquirer 210. It is inappropriate for the doctor to make a diagnosis of an arrhythmia, solely based on such insufficient information. For the doctor to make a diagnosis, a more detailed display is needed, such as displaying the pulse wave information itself or displaying each value of the pulse rate and the pulse wave information in a sufficiently identifiable form, for example.

In the embodiment, since the result of the determination on whether it is a pulse wave abnormality period or not is acquired in the first analysis result information, as described above, representative information during the pulse wave abnormality period may be used as more detailed information. Thus, information with relatively high importance in diagnosing an arrhythmia can be viewed in detail.

For example, as shown in FIG. 8, the second analysis result information may be information which displays changes with time in the body motion information (acceleration information), the pulse waveform and the pulse interval waveform over a relatively short period of approximately 20 seconds. As shown in FIG. 8, as the display target section is limited to approximately 20 seconds, changes in the acceleration waveform, the pulse waveform and the pulse interval waveform can be displayed in detail (without thinning or the like). The section within the pulse wave abnormality period displayed via the second analysis result information is not limited to 20 seconds and may be 60 seconds or the like. Various modifications are possible.

As shown in the example of displaying the acceleration waveform in FIG. 8, the processor 230 may generate information for displaying at least one of the body motion information and the wearing state information during a predetermined section in the pulse wave abnormality period, as the second analysis result information. In the first analysis result information, it is assumed that section reliability is used as the reliability index. The section reliability is determined, for example, with respect to a predetermined 20-second section or the like. Details of the section reliability will be described later in the explanation of a section reliability determination device 227.

That is, in the first analysis result information, it is assumed that the reliability index of pulse wave measurement is displayed on a 20-second basis, and displaying details of the body motion information and the wearing state information used when finding the reliability index (for example, changes in time series during the 20 seconds) or the like is not particularly considered. However, with respect to the determination on whether the pulse wave measurement during the target section is reliable or not, it is desirable that the viewer (doctor) checks the result of the determination by the biological information processing system 200, instead of directly using the result of the determination.

That is, in the second analysis result information, though displaying the result of the determination by the section reliability determination device 227 is not precluded, more detailed information used for reliability determination may be displayed. Specifically, the waveform of the body motion information itself or the waveform of the wearing state index value itself may be displayed. For the determination of the wearing state, the signal level of the pulse wave information may be used. Therefore, the pulse waveform shown in FIG. 8 and the like is information that can have both a first aspect as detailed information for diagnosing the patient's arrhythmia and a second aspect as detailed information for determining reliability.

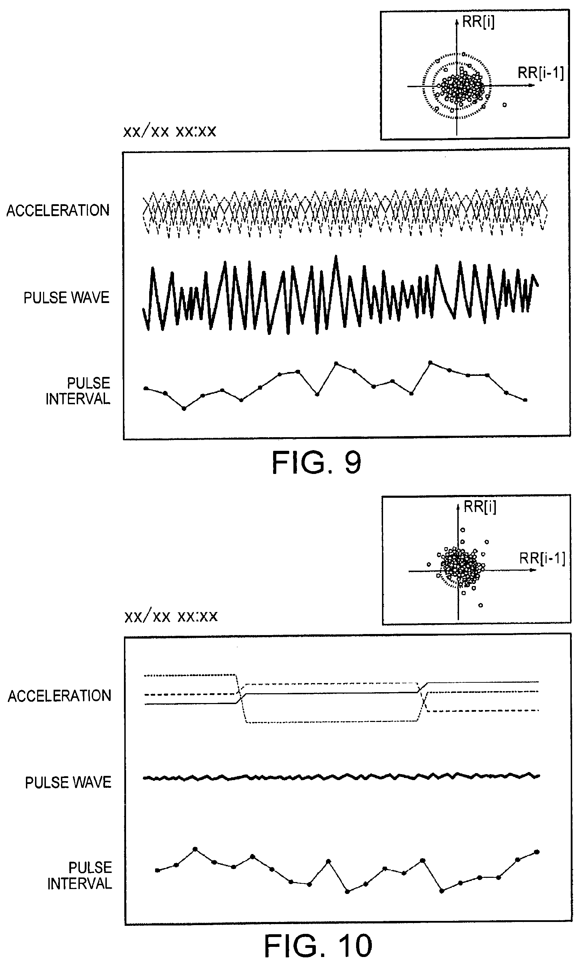

Other examples of the second analysis result information are shown in FIGS. 9 and 10. In FIG. 9, from the acceleration waveform displayed via the second analysis result information, it can be understood that the variation in value is large. Therefore, the influence of the body motion noise can be suspected. In FIG. 10, from the wearing state information (pulse waveform) displayed via the second analysis result information, it can be understood that the signal level is very low. Therefore, the influence of wearing failure can be suspected.

Alternatively, the analysis result information described above with reference to FIGS. 4A to 6B may be displayed along with the body motion information (acceleration information), the pulse waveform and the pulse interval waveform, as shown in FIGS. 8 to 10. In this case, the analysis result information of FIG. 4A and the like may be prepared from a part or all of the information corresponding to the target section (for example, 20 seconds) of the second analysis result information. Thus, the reliability index of measurement of the pulse wave information can be displayed in the intelligible form as described above.

Since the arrhythmia can occur a plurality of times (a plurality of episodes of arrhythmia can occur) during a period of three to ten days, the pulse wave abnormality period, too, can be detected a plurality of times during the measurement period, as shown in the first analysis result information of FIG. 7. In this case, in order for the doctor to make a proper diagnosis with respect to each pulse wave abnormality period, at least one section may be set in each pulse wave abnormality period and the display via the second analysis result information may be carried out, targeting this section.

That is, the processor 230 generates second analysis result information that can display at least one episode waveform with respect to one pulse wave abnormality period in which an episode of arrhythmia (continuous occurrence of arrhythmia) is suspected. The episode waveform in this example is information showing a representative waveform during a pulse wave abnormality period in which an episode of arrhythmia is suspected. The episode waveform corresponds to each of FIGS. 8 to 10. Consequently, the second analysis result information is information for displaying a plurality of episode waveforms.

In this case, the plurality of episode waveforms may be displayed, arranged in time series order, and the doctor may view the episode waveforms in the order of detection of the pulse wave abnormality periods. However, in the embodiment, the calculation of the reliability index is carried out. Therefore, with respect to each of the plurality of episode waveforms, how high the reliability of the pulse wave measurement is can be estimated.

With an episode waveform with high reliability, the detection accuracy of the pulse wave abnormality period is expected to be high. Therefore, there is a high possibility that the pulse wave abnormality period is detected because of an abnormality in the patient. For the doctor, viewing this episode waveform is useful for diagnosing an arrhythmia. Meanwhile, with an episode waveform with low reliability, there is a possibility that the pulse wave abnormality period is detected because of a noise even though the patient has no abnormality (false positive). Therefore, viewing this episode waveform might be useless for diagnosing an arrhythmia.

If the second analysis result information includes a plurality of episode waveforms, the degree of priority in display may be differentiated among these episode waveforms according to their reliability, instead of handling them equally. Specifically, if the reliability index for a first pulse wave abnormality period is higher than the reliability index for a second pulse wave abnormality period, the processor 230 generates second analysis result information in such a way that the analysis result information corresponding to the first pulse wave abnormality period is preferentially displayed over the analysis result information corresponding to the second pulse wave abnormality period.

Thus, the analysis result information (episode waveform) corresponding to the first pulse wave abnormality period, which has higher reliability and in which it is suspected that the patient truly has an arrhythmia, is displayed preferentially. Therefore, the doctor can preferentially view useful information for diagnosing the arrhythmia. In contrast, the analysis result information (episode waveform) corresponding to the second pulse wave abnormality period, which has lower reliability and in which a false positive is suspected, has a lower degree of priority. Therefore, the doctor can leave until later the viewing of information that can be useless for diagnosing the arrhythmia, or the like.

In the description below, an analysis report will be used as an example of the output of the biological information processing system 200 including the first and second analysis result information. Although the timing of preparation, the frequency of preparation and the like of the analysis report can be implemented with various modifications, in this example, a report showing a result corresponding to one round of test, that is, a report prepared by storing data over a period of about three to ten days, will be described. In this case, "differentiating the degree of priority among a plurality of episode waveforms included in the second analysis result information" can be achieved by differentiating the display form of the episode waveform in the analysis report.

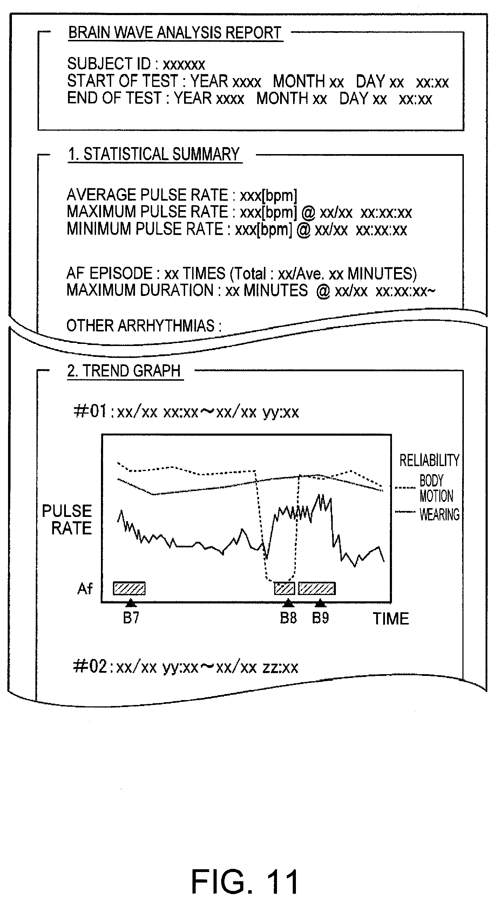

FIGS. 11 and 12 show an example of the analysis report in the embodiment. The analysis report is made up of four items, that is, statistical summary, trend graph, episode waveform, and full waveform.

The statistical summary is an item showing a representative statistical quantity indicating the state of the user (patient) during the testing period. For example, an average value, maximum value, minimum value and the like of pulse rate are displayed, as shown in FIG. 11. The maximum pulse rate and the minimum pulse rate may be displayed along with the timings (month, day, hour, minute, second) when these pulse rates are detected, as shown in FIG. 11. Also, the number of times it is determined that atrial fibrillation (Af) has occurred, total duration, average duration per occurrence, maximum duration and the like are displayed. The maximum duration may be displayed along with the timing of occurrence (occurrence start timing, end timing) of this atrial fibrillation.

The full waveform shows the acceleration waveform, the pulse waveform, the pulse interval waveform and the state of occurrence of atrial fibrillation (result of determination of pulse wave abnormality period) over the entire testing period, as shown in FIG. 12.

As can be understood from FIG. 11, the statistical summary is suitable for grasping the summary of the testing period in the form of numerical values, but is unsuitable for grasping changes in time series in the state of the user because no specific waveform or the like is displayed. Therefore, more detailed information is necessary for the doctor to make a diagnosis. Also, as can be understood from FIG. 12, the full waveform displays detailed information but results in an excessively large amount of information. Therefore, properly summarized information or important information extracted from the whole needs to be displayed for the doctor to make a diagnosis.

As information that facilitates the diagnosis by the doctor, the first analysis result information and the second analysis result information are generated in the embodiment, as described above. The first analysis result information in the embodiment corresponds to the trend graph in FIG. 11, for example. The second analysis result information corresponds to the episode waveform in FIG. 12, for example.

Specific examples of the technique for preferentially displaying the analysis result information (episode waveform) corresponding to a predetermined pulse wave abnormality period over other analysis result information are conceivable. For example, the processor 230 may generate the second analysis result information in such a way that the analysis result information corresponding to the first pulse wave abnormality period is displayed earlier than the analysis result information corresponding to the second pulse wave abnormality period.

In the case of outputting a report on a paper medium, pdf file or the like as the output including the first and second analysis result information (for example, analysis report), this analysis report shows a plurality of items in a fixed order, and no change or the like is made to the order. An item which comes earlier in the order of display is displayed at a position closer to the beginning (first page), whereas an item which comes later in the order of display is displayed at a position closer to the end (last page). The page order in which the analysis report is viewed varies depending on the viewer. However, it is generally expected that viewing starts with an item close to the first page. That is, "being displayed earlier" in this example may be being arranged at a position closer to the first page.

The first analysis result information (trend graph) in FIG. 11 corresponds to FIG. 7. In this example, as can be understood from the graph of the reliability index, the reliability corresponding to a section B7 is high, whereas the reliability corresponding to a section B8 is low. In this case, the episode waveform corresponding to B7 (corresponding to FIG. 8) is displayed as in C1 in FIG. 12, and the episode waveform corresponding to B8 (corresponding to FIG. 9) is displayed as in C2 in FIG. 12. That is, the episode waveform corresponding to B7 with high reliability is displayed at a position closer to the first page. Also, the episode waveform corresponding to FIG. 10 has low reliability and therefore may be displayed more to the back (subordinately) as in C3 in FIG. 12. As can be understood from FIG. 12, in this example, an acceleration waveform, a pulse waveform and a pulse interval waveform are displayed as episode waveforms. Specifically, as the episode waveforms in FIG. 12, the information formed by omitting the analysis result information corresponding to FIGS. 4A to 6B from FIGS. 8 to 10 is used.

Alternatively, each kind of information that is a display target may be associated with information indicating the display order. For example, the analysis result information corresponding to the first pulse wave abnormality period may be provided with first internal data (first metadata) indicating the display order, and the analysis result information corresponding to the second pulse wave abnormality period may be provided with second internal data (second metadata) indicating the display order. The data indicating the display order is provided in a format that enables comparison between data (for example, numerical data that can be determined as large or small). Thus, by comparing the first internal data with the second internal data, which comes earlier in the order can be determined. For example, if the internal data is numerical data and the position in the order of display becomes earlier as the numerical value becomes smaller, two internal data are generated in such a way that the first internal data < the second internal data, and each of these is provided for the analysis result information corresponding to each pulse wave abnormality period. This enables differentiation in the degree of priority. In the case of deciding the order of display using such internal data, processing of generating display images that are put in order on the basis of the internal data may be performed by the processor 230 according to the embodiment or may be performed by a processor in a device having a display. Also, the internal data may be provided for other types of information such as the first analysis result information as well.

It is conceivable that the viewer follows the procedure of grasping the summary over the entire measurement period from the first analysis result information and then making a detailed diagnosis using the second analysis result information. That is, when a display area for displaying the second analysis result information is set, the degree of priority of the viewing by the viewer is considered to be higher as the display position (arrangement position) in the display area is closer to the first analysis result information. That is, "being displayed earlier" may be being arranged at a position closer to the first analysis result information.

Also, "being preferentially displayed" may be based on whether the display area (display size) is large or small. The second analysis result information may be generated in such a way that the analysis result information corresponding to the second pulse wave abnormality period is displayed in a reduced size, compared with the analysis result information corresponding to the first pulse wave abnormality period. Thus, since the episode waveform with high reliability is displayed in a relatively large size and the episode waveform with low reliability is displayed in a relatively small size, the user can easily understand which episode waveform the user should pay attention to.

In the example of FIG. 12, the episode waveform with low reliability (C2 and C3 in FIG. 12, corresponding to FIGS. 9 and 10) is reduced in size to 1/4 of the episode waveform with high reliability (C1 in FIG. 12, corresponding to FIG. 8), and the visibility of the episode waveform with high reliability is relatively high.

The method for "preferentially displaying" is not limited to whether the position in the order is earlier or later, or whether the display size is large or small, and may be implemented by various kinds of highlighting processing such as changing the background color or changing the color or size of letters.

Up to this point, the description is based on the case where detailed information corresponding to a predetermined section in a pulse wave abnormality period, that is, only an episode waveform, is displayed. However, this example is not limiting. For instance, in some cases, depending on the objective of diagnosis or the person making a diagnosis, it is demanded that a space for showing a representative non-episode waveform (detailed information corresponding to a section that is not a pulse wave abnormality period) should be added to the analysis report.

In such a case, the analysis result information for displaying the body motion waveform, the pulse waveform and the pulse interval waveform may be generated, for example, similarly to FIG. 8 or the like, targeting a section that is not a pulse wave abnormality period. In this case, instead of handling the respective non-episode waveforms equally, the degree of priority corresponding to the reliability may be set, or a non-episode waveform with high reliability may be displayed earlier or displayed in a relative large size.

Also, while it is assumed in the above description that the output including the first and second analysis result information is in a static format, the output according to the embodiment may be in a dynamic (interactive) format. For example, a pulse wave abnormality period may be designated on the first analysis result information (trend graph), so that a representative waveform (second analysis result information, episode waveform) corresponding to the designated pulse wave abnormality period will be displayed. The display of the representative waveform in this example may be opening a new display window or may be jumping to a display site. Other formats may also be employed. In this case, since the concept of whether the position in the order of display is earlier or later is insubstantial, highlighting such as changing the background color may be carried out as the method for "preferentially displaying".

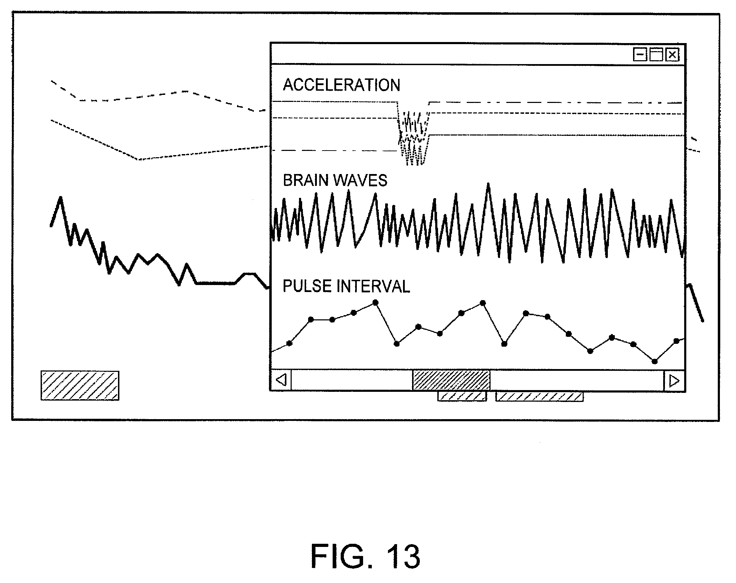

An example of display of the first and second analysis result information in a dynamic format is shown in FIG. 13. FIG. 13 shows an example where a representative waveform is displayed by opening a new display window. In FIG. 13, it is assumed that the pulse wave abnormality period corresponding to B4 (B7) in FIG. 7 is designated in the state where the trend graph shown in FIG. 7 is displayed. A new display window (popup screen) is superimposed on the trend graph of FIG. 7. In the new display window, the representative waveform corresponding to FIG. 8 is displayed. Also, the display of the representative waveform is not limited to the fixed display of a waveform corresponding to a section with a predetermined length (for example, 20 seconds), and the transition of the waveform with time before and after the section may be displayed. For example, in FIG. 13, a scroll bar is provided at the bottom of the new display window, and the user can change the section to be the waveform display target by operating the scroll bar. For example, the user can cause the waveform that is earlier in time series to be displayed by pressing the left arrow button in the scroll bar in FIG. 13 or moving the scroll box to the left. The user can cause the waveform that is later in time series to be displayed by pressing the right arrow button or moving the scroll box to the right. The user interface in the case of viewing the transition of pulse waves with time before and after the displayed representative waveform is not limited to the scroll bar and can be implemented with various modifications.

In the example described above, the information shown in FIG. 7 is displayed as the first analysis result information and the information formed by omitting the analysis result information shown in FIGS. 4A to 6B from FIGS. 8 to 10 is displayed as the second analysis result information in the analysis report. However, this example is not limiting. As described above, the first analysis result information may be changed to FIGS. 2 and 3. Alternatively, the information of FIGS. 8 to 10 itself, without omitting the analysis result information shown in FIGS. 4A to 6B, may be displayed as the second analysis result information. Alternatively, the analysis result information shown in FIGS. 4A to 6B may be displayed as independent items. Alternatively, if the format used is capable of displaying dynamic images, changes in time series in the analysis result information shown in FIG. 4A or the like may be displayed as a dynamic image.

### 4. Example of System Configuration

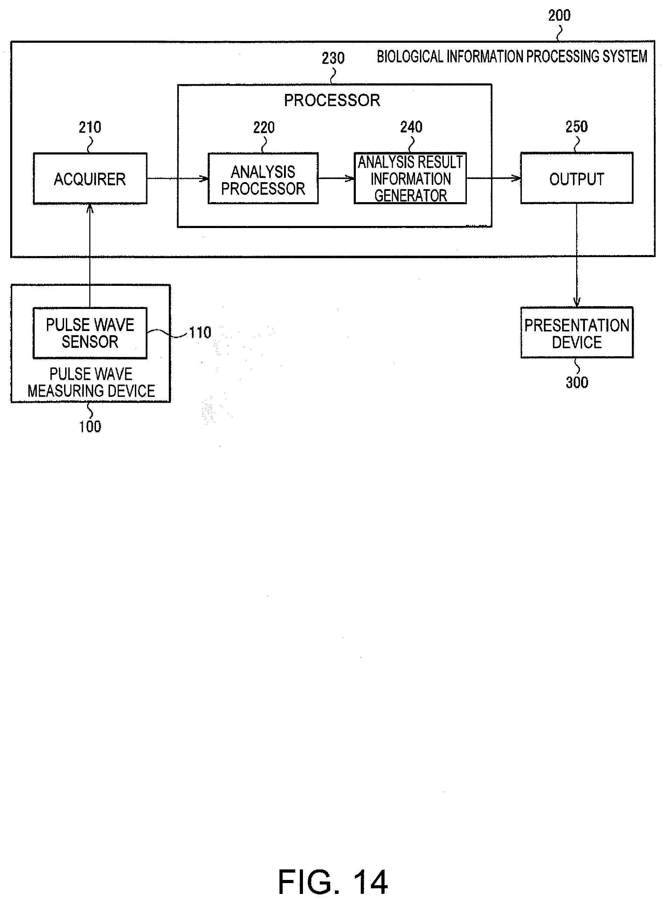

Next, an example of the configuration of the biological information processing system 200 or the like according to the embodiment will be described. First, a specific example of the system configuration of the biological information processing system 200 will be described with reference to FIG. 14 and the like. Subsequently, an example of the appearance of a pulse wave measuring device 100 and a specific example of a system including the biological information processing system 200 will be described with reference to FIGS. 19A to 21.

### 4.1 Example of Configuration of Biological Information Processing System

As shown in FIG. 1, the biological information processing system 200 according to the embodiment includes the acquirer 210, the processor 230, and the output 250.

The acquirer 210 acquires pulse wave information. For example, in the case where the biological information processing system 200 according to the embodiment is implemented by a server system and the server system acquires pulse wave information from the pulse wave measuring device 100 (biological information detection device) worn by the user, as described later with reference to FIG. 21A, the acquirer 210 may be a communication device which communicates with the pulse wave measuring device via a network (a receiver which receives information from the pulse wave measuring device).

In this case, the acquirer 210 acquires sensor information from a pulse wave sensor included in the pulse wave measuring device. Here, the pulse wave sensor is a sensor for detecting a pulse wave signal and may be, for example, a photoelectric sensor including a light emitter and a light receiver. It is known that the pulse wave sensor can be implemented by various sensors such as a photoelectric sensor and other types of sensors (for example, ultrasonic sensor). These sensors can be broadly applied as the pulse wave sensor in the embodiment.

The processor 230 performs analysis processing on atrial fibrillation on the basis of the pulse wave information acquired by the acquirer 210 and generates analysis result information on the basis of the result of the analysis. The functions of the processor 230 can be implemented by hardware such as various processors (CPU or the like) or ASIC (gate array or the like), or by a program or the like.

The output 250 outputs the analysis result information generated by the processor 230. Various output forms from the output 250 are possible. For example, the analysis result information may be transmitted to another device via a network. In this case, the output 250 is implemented by a communication device. The network in this case can be implemented by a WAN (wide area network), LAN (local area network) or the like, and may be wired or wireless. Alternatively, the output 250 may print the analysis result information. In this case, the viewer views the printed analysis result information on a paper medium or the like.

The processing by the processor 230 of generating the analysis result information for displaying various kinds of information may be processing of generating display screen information or may be processing of generating data for printing. Information for screen display (for example, an HTML file or the like prescribing the arrangement relation between the respective kinds of information) may be used. Generating the information for screen display may be, for example, processing of generating a page for viewing (for example, a web page).

The output 250 may be a display processor which displays a display screen prescribed by the display screen information, on the display of a terminal device. For example, a device including the biological information processing system 200 according to the embodiment may have a display, and the display screen may be displayed on this display. Or, the display screen may be displayed on the display of a terminal device which is different from the device including the biological information processing system 200. Alternatively, the output 250 may transmit the data for printing to a printing device. Alternatively, the output 250 may be a transmitter (communication device) which transmits the information for screen display in response to a request from a terminal device which is different from the device including the biological information processing system 200. In this case, a specific screen display using the information for screen display may be carried out on the side of the terminal device which has receives this information for screen display. As an example, in the case where a viewing request for a web page using a web browser is sent from a terminal device to the biological information processing system 200, the processor 230 may send back information of the web page corresponding to the viewing request, and the web browser of the terminal device may interpret the information of this web page and display the information on the display. However, the transmission and reception of information between the terminal device and the biological information processing system 200 is not limited to the transmission and reception using the web browser. For example, a native application may be used.

FIG. 14 shows a detailed example of the configuration of the system including the biological information processing system 200. However, the biological information processing system 200 is not limited to the configuration of FIG. 14 and can be implemented with various modifications such as omitting a part of the components or adding another component. In the example of FIG. 14, the acquirer 210 of the biological information processing system 200 acquires pulse wave information from the pulse wave measuring device 100 including a pulse wave sensor 110.

The processor 230 includes an analysis processor 220 which performs analysis processing on an arrhythmia (atrial fibrillation), and an analysis result information generator 240 which generates analysis result information.

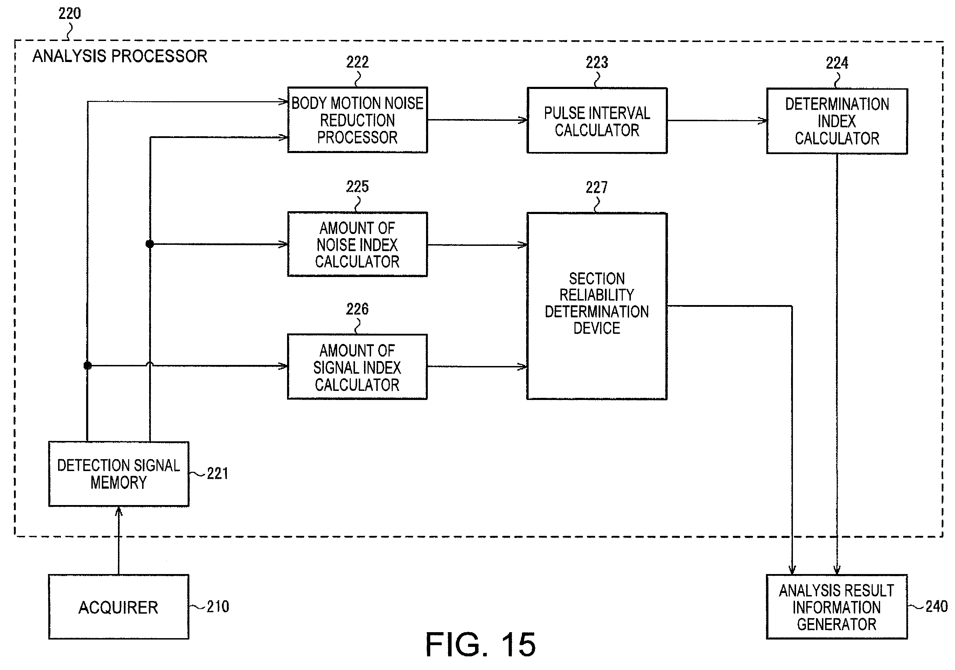

FIG. 15 shows a detailed example of the configuration of the analysis processor 220. As shown in FIG. 15, the analysis processor 220 includes a detection signal memory 221, a body motion noise reduction processor 222, a pulse interval calculator 223, a determination index calculator 224, an amount of noise calculator 225, an amount of signal calculator 226, and a section reliability determination device 227.

The detection signal memory 221 stores the information acquired by the acquirer 210. Here, it is assumed that the acquirer 210 acquires body motion information (in a narrow sense, acceleration information) indicating the body motion of the user, as well as the pulse wave information. The detection signal memory 221 stores the pulse wave information and the body motion information. For example, the body motion information is information from an acceleration sensor included in the pulse wave measuring device 100.

The body motion noise reduction processor 222 acquires a pulse wave signal and a body motion signal from the detection signal memory 221 and performs processing of reducing the body motion noise on the pulse wave signal. The body motion noise reduction processor 222 can be implemented, for example, by adaptive filter processing or the like. In the embodiment, as described later, since the reliability index is used, even if the body motion noise is not eliminated and consequently an arrhythmia is erroneously detected (a false positive is detected), it is possible to lower the degree of priority of display for the result of this detection. Therefore, the body motion noise reduction processor 222 in the embodiment is not an essential component and can be omitted.

The pulse interval calculator 223 finds the pulse interval on the basis of the pulse wave information. If the influence of the noise on the pulse waveform is small, the peak interval or the like in the pulse waveform can be used as the pulse interval. However, the pulse waveform is usually not such a smooth and regular waveform. Therefore, the pulse interval may be found by frequency analysis processing on pulse wave information corresponding to a certain period. As an example, the pulse interval calculator 223 slices out, at each sampling, a frame of the pulse wave information with the body motion noise component reduced by the body motion noise reduction processor 222, and calculates a frequency spectrum by short-time frequency analysis (STFT (short-time Fourier transform) analysis). Then, the pulse interval calculator 223 calculates a parameter equivalent to the pulse interval for each frame on the basis of the frequency spectrum that is calculated.

The frame in this example may be, for example, a period of about four seconds. In the embodiment, the frame of four seconds or the like for which the pulse interval is found is also referred to as a pulse interval calculation period. In the description below, the pulse interval calculation period is a period of four seconds and this period is shifted by one second each, so as to find the pulse interval once very second. However, the length of the pulse interval calculation period and the rate of finding the pulse interval can be implemented with various modifications. The pulse interval that is found is outputted to the determination index calculator 224.

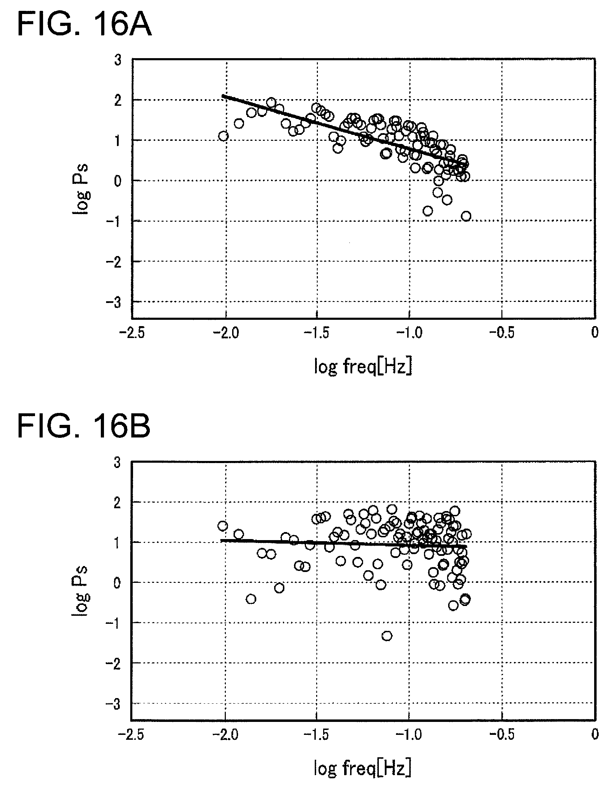

The determination index calculator 224 finds a determination index for determining whether an arrhythmia (atrial fibrillation) has occurred or not, specifically, whether it is a pulse wave abnormality period or not, on the basis of the pulse interval. FIGS. 16A and 16B are graphs showing the result of performing frequency analysis in a band from 0.01 Hz to 0.2 Hz is performed for one frame of a waveform signal indicating a variation in the electrocardiogram RR interval and then converting the peak frequency and power into logarithms. FIG. 16A is a graph in the case where atrial fibrillation has not occurred. FIG. 16B is a graph in the case where atrial fibrillation has occurred.

As can be understood from FIGS. 16A and 16B, when the electrocardiogram RR interval is used, the intercept and slope of the regression line changes according to whether atrial fibrillation has occurred or not. Therefore, it is possible to determine whether atrial fibrillation has occurred or not, from the intercept and slope. Thus, the determination index calculator 224 may find a graph showing the peak frequency and power converted into logarithms with respect to the pulse waveform showing the fluctuation in the pulse interval, and may find the intercept and slope of the graph as the determination index. By comparing the determination index with the intercept or the like in a normal state and the intercept or the like at the occurrence of atrial fibrillation, it is possible to determine whether it is a pulse wave abnormality period in which an abnormality is observed in the pulse wave information or not, specifically, whether atrial fibrillation has occurred or not. The determination index calculator 224 may output the result of determination based on the intercept or the like (result of determination on whether atrial fibrillation has occurred or not), instead of using the intercept and slope as index values.

Alternatively, the power in a part of the frequency band may be calculated, of the change with time in the pulse interval. When atrial fibrillation has occurred, the power increases by several times, showing a significant difference, compared with when atrial fibrillation has not occurred. Therefore, whether atrial fibrillation has occurred or not may be determined on the basis of the change in power.

It is known that the fluctuation in time series in the pulse interval increases at the occurrence of atrial fibrillation. Therefore, modifications such as finding a statistical quantity such as a variance value or standard deviation may be carried out. Also, in such a case, a statistical quantity of the amount of change (difference or proportion) from the pulse interval at the immediately preceding timing may be found, instead of the statistical quantity of the pulse interval itself. In this way, the processing by the determination index calculator 224 can be implemented with various modifications. In the description below, it is assumed that the result of determination on whether it is a pulse wave abnormality period or not is outputted from the determination index calculator 224.

The amount of noise calculator 225 finds an amount of noise index indicating the degree of noise included in the pulse wave information. Specifically, the amount of noise calculator 225 may acquire the body motion information (acceleration information) from the detection signal memory 221 and find an index value indicating the degree of the body motion noise. More specifically, as the amount of body motion noise, the power in a predetermined band of the amounts of accelerations on the three axes of the acceleration sensor can be used.

The amount of signal calculator 226 finds an amount of signal index indicating the amount of signal (signal level) included in the pulse wave information. The amount of signal calculator 226 may simply find the amplitude level (amount of effective signal or the like) of the pulse wave signal, as the amount of signal index. The analysis processor 220 is not limited to the configuration including both the amount of noise calculator 225 and the amount of signal calculator 226 and may be configured to include one of these calculators.

The processor 230 (in a narrow sense, the analysis processor 220 of the processor 230) also includes the section reliability determination device 227 which finds the reliability index corresponding to each section in the measurement period on the basis of at least one of the body motion information and the pulse wave information of the user.

The section reliability determination device 227 can find the reliability index based on the amount of noise index, for example, by a method such as extracting, with a digital filter, a predetermined band and finding an effective value from the acceleration waveform on each axis of the acceleration sensor, then multiplying the value by a coefficient corresponding to the degree of influence on pulse measurement with respect to each axis, and adding the results. The predetermined band in this example is, for example, 0.5 to 2 [Hz]. This corresponds to a band which can overlap with the frequency band of pulse. As the reliability index based on the amount of signal index, the amount of effective signal of the pulse wave signal may be used.

The length of each section in the measurement period to be the target of the section reliability determination in this example is, for example, approximately 20 seconds. In this case, the section reliability determination device 227 determines the reliability index (section reliability) corresponding to the period of 20 seconds on the basis of the amount of effective signal of the body motion information (acceleration signal) during the 20 seconds outputted from the amount of noise calculator 225 and the amount of effective signal of the pulse wave information during the 20 seconds outputted from the amount of signal calculator 226.

The analysis result information generator 240 of the processor 230 generates analysis result information on the basis of the determination index outputted from the determination index calculator 224 and the reliability index outputted from the section reliability determination device 227. Details of the analysis result information are as described above.

In the example of FIG. 14, the output 250 outputs the analysis result information to another presentation device 300. That is, FIG. 14 shows an example in which the analysis result information is transmitted via a network or the like, as described above. The presentation device 300 presents the analysis result information acquired from the output 250, to the viewer. In a narrow sense, the presentation device 300 may include a display and may display the analysis result information on the display.

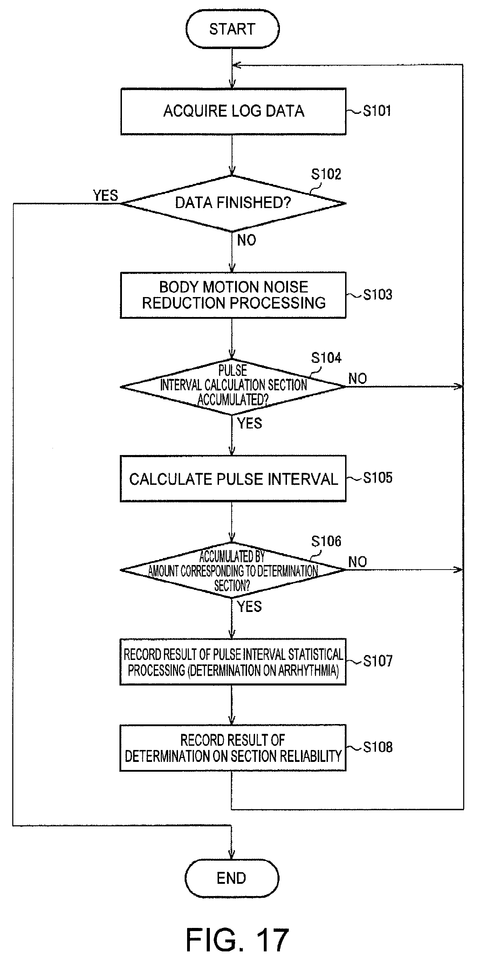

FIG. 17 shows a flowchart explaining the processing carried out by the analysis processor 220 in the biological information processing system 200 according to the embodiment. As this processing is started, first, log data acquired by the acquirer 210 is read (S101). The log data in this example is, for example, data of pulse wave information and body motion information accumulated over one entire measurement period of three to ten days. In S101, processing of reading one piece of data from among the entire data may be carried out.

Next, whether the processing on the log data is finished or not is determined (S102). For example, the processing may be performed sequentially from the beginning of the log data, and whether unprocessed data is left or not may be determined. If there is no unprocessed data (No in S102), the processing by the analysis processor 220 ends and the flow shifts to processing of generating analysis result information for display based on the result of the analysis.

If there is unprocessed data (Yes in S102), the processing of S103 and onward is performed. Specifically, first, the body motion noise reduction processor 222 performs processing of reducing the body motion noise (S103). The body motion noise reduction processing can be implemented, for example, by filter processing as described above.

Then, it is determined whether the pulse information after the reduction of the body motion noise is accumulated by the amount corresponding to the pulse interval calculation section or not (S104). Since the pulse interval calculation section is, for example, four seconds, the determination in S104 results in Yes when information corresponding to four seconds is accumulated. For example, if the sampling rate of the pulse wave information is 16 Hz, the information corresponding to four seconds is 64 pieces of data. If the result is No in S104, the flow returns to S101 to read the next log data. The pulse interval calculation section may be set with a shift of one second, as described above. In such a case, in the processing of S104, data corresponding to four seconds need not be accumulated from scratch, and already accumulated data may be utilized as data corresponding to three seconds. That is, in S104, the determination results in Yes once every second.

If it is Yes in S104, the pulse interval is calculated on the basis of the accumulated pulse wave information corresponding to the pulse interval calculation section (S105). The specific calculation method is as described above. The processing of S105 is executed at the same rate as the rate at which the determination results in Yes in S104. Therefore, the pulse interval is found, for example, one pulse interval per second.

Next, it is determined whether data is accumulated by the amount corresponding to a determination section for which reliability is determined (S106). The determination section in this example is, for example, 20 seconds. Therefore, in S106, the determination results in Yes once every 20 seconds, and otherwise it is No. In the case of No, the flow returns to S101 to read the next log data.

If it is Yes in S106, first, whether it is a pulse wave abnormality period or not is determined on the basis of the pulse intervals accumulated by the amount corresponding to the determination section (S107). Since the pulse interval is found, for example, once every second, the determination index calculator 224 may carry out this determination using changes in time series about 20 pulse intervals (21 pulse intervals as shown in FIG. 8 or the like, depending on the handling of the pulse intervals at the ends of the determination section).

The section reliability determination device 227 determines the section reliability on the basis of the pulse wave information and the body motion information corresponding to 20 seconds (S108). After the processing of S108, the flow returns to S101 to read the next log data.

By carrying out the processing shown in FIG. 17, the pulse interval can be found once every second, and the result of determination on the pulse wave abnormality period and the section reliability can be found once every 20 seconds. Also, since the pulse interval can be converted to find the pulse rate, the pulse rate, too, can be found at the rate of once every second.

The method in which the pulse interval is calculated once every second by setting the pulse interval calculation section of four seconds with a shift of one second, that is, allowing an overlap of three seconds, is described above, and similar processing can be applied to the determination section for determining a pulse wave abnormality period. In the flowchart of FIG. 17, an example where a determination section of 20 seconds is used at the time of finding the result of determination is shown and it is assumed that the result of determination is found once every 20 seconds. However, if the determination section is set with a shift of x seconds each, the result of determination can be found once every x seconds. In this example, x seconds may be one second as in the calculation of the pulse interval, or may be such lengths as two seconds, five seconds, or ten seconds.

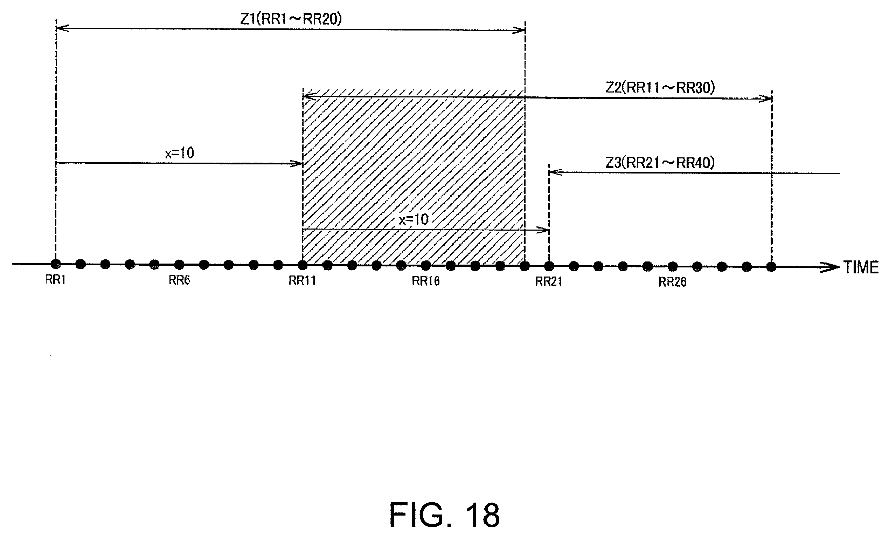

In the case where overlaps of determination sections are thus allowed, the value of one pulse interval corresponds to a plurality of determination sections. A specific example is shown in FIG. 18. In FIG. 18, it is assumed that the determination section is 20 seconds, the value x is ten seconds, and the pulse interval is calculated once every second. In this example, the value of the pulse interval at the end point of the determination section is not used in this determination section. Therefore, 20 values of the pulse interval are included in the determination section of 20 seconds. In FIG. 18, a first determination section Z1 and a second determination section Z2 next to Z1 are set. Of the pulse intervals, RR11 to RR20 are included in both Z1 and Z2. That is, a result of determination R1 on a pulse wave abnormality period found from Z1, and a result of determination R2 on a pulse wave abnormality period found from Z2 are both information based on RR11 to RR20.

In this case, as the result of determination on a pulse wave abnormality period based on RR11 to RR20, one of R1 and R2 may be used. However, since a plurality of pieces of information corresponding to RR11 to RR20 is found, the plurality of pieces of information may be combined together. As an example, the result of determination in the section corresponding to RR11 to RR20 may be decided, using the logical disjunction, logical conjunction, majority rule or the like of R1 and R2. While x=10 holds in FIG. 18, if x is smaller than that, one pulse interval value corresponds to a greater number of determination sections. For example, if x=1 holds, one pulse interval value corresponds to 20 determination sections. Therefore, the result of determination for this one pulse interval may be found using the logical disjunction, logical conduction, majority rule or the like of 20 patterns of results of determination. The length of the determination section and the length of x may be implemented with other modifications, as described above.

The feature that the determination section may be set with a shift of x seconds each similarly applies to the case of finding the reliability index (section reliability). In this case, similar settings need not be carried out for the result of determination on a pulse wave abnormality period and for the section reliability. Therefore, the length of the determination section, or x, which is the width by which the determination section is shifted at the time of setting, may be set to different values.

### 4.2 Specific Example of Pulse Wave Measuring Device and the Like

FIGS. 19A to 20 show an example of the appearance of the pulse wave measuring device 100 (wearable device) for gathering pulse wave information. The wearable device in the embodiment has a band 10, a case 30, and a sensor 40. The case 30 is attached to the band 10. The sensor 40 is attached to the case 30.

The band 10 is to be wound on the user's wrist so that the user can wear the wearable device. The band 10 has a band hole 12 and a buckle 14. The buckle 14 has a band insertion part 15 and a protrusion 16. The user inserts one end of the band 10 into the band insertion part 15 of the buckle 14 and inserts the protrusion 16 of the buckle 14 into the band hole 12 of the band 10, thus wearing the wearable device on the wrist. The band 10 may also be configured with a clasp instead of the buckle 14.

The case 30 is equivalent to the main body of the wearable device. Inside the case 30, various components of the wearable device such as the sensor 40 and a circuit board or the like, not shown, are provided. That is, the case 30 is a casing accommodating these components.

The case 30 is provided with a light emitting window 32. The light emitting window 32 is formed of a light-transmitting member. The case 30 provided with a light emitting device as an interface mounted on a flexible substrate. The light from the light emitting device is emitted out of the case 30 via the light emitting window 32.

The wearable device is worn by the user on the wrist, as shown in FIG. 21A, and pulse wave information (in a broad sense, biological information) is measured in the state where the wearable device is worn.

Next, an example of a specific device which implements the biological information processing system 200 according to the embodiment will be described. The biological information processing system 200 according to the embodiment may be, for example, a server system. An example of this case is shown in FIG. 21A. The biological information processing system 200 as a server system is connected to the pulse wave measuring device 100 via a network NE and acquires pulse wave information from the pulse wave measuring device 100. Since the wearable device (pulse wave measuring device 100) worn by the user needs to be small-sized and lightweight, the battery capacity, the processing capability of the processor inside the device, or the memory capacity for data is very limited. In contrast, in the server system, since resources are less limited, the processing of analyzing the pulse wave information and the processing of generating the analysis result information can be carried out at high speeds, and more data (pulse wave information or analysis result information) can be held.

It suffices that the biological information processing system 200 can acquire the pulse wave information gathered by the pulse wave measuring device 100. Therefore, the biological information processing system 200 is not limited to being connected directly to the pulse wave measuring device 100. For example, the pulse wave measuring device 100 may be connected to another processing device 400, and the biological information processing system 200 may be connected to the processing device 400 via the network NE, as shown in FIG. 21B. The processing device 400 in the case may be, for example, a mobile terminal device such as a smartphone used by a user wearing the pulse wave measuring device 100. For the connection between the pulse wave measuring device 100 and the processing device 400, an equivalent to the network NE may be used, and near field wireless communication or the like may also be used.

The biological information processing system 200 according to the embodiment may be implemented by a processing device such as a smartphone (in a narrow sense, a mobile terminal device), instead of the server system. An example of the configuration in this case is shown in FIG. 21C. The mobile terminal device such as a smartphone often has limitations to its processing capability, memory area and battery capacity, compared with the server system. However, in consideration of the recent improvement in performance, it may be possible to secure sufficient processing capability and the like. Therefore, if the requirements of processing capability and the like are met, a smartphone or the like can be used as the biological information processing system 200 according to the embodiment, as shown in FIG. 21C.

Moreover, when improvement in terminal performance or the way of using the device or the like is considered, an embodiment in which the pulse wave measuring device 100 includes the biological information processing system 200 according to the embodiment is not precluded. In this case, the acquirer 210 receives (acquires) information from the pulse wave sensor 110 in the same device. In the case where the biological information processing system 200 is installed in the pulse wave measuring device 100, this biological information processing system 200 hardly needs to perform analysis, saving or the like of data with respect to a large number of users, and may only have to handle one or a smaller number of user using the pulse wave measuring device 100. That is, it is highly possible that, even with the processing capability of the pulse wave measuring device 100, requests from users can be dealt with.

That is, the technique in the embodiment can be applied to a biological information processing device (biological information analysis device, biological information measuring device, biological information detecting device) including an acquirer 210 which acquires pulse wave information, a processor 230 which performs analysis processing on the pulse wave information to generate analysis result information, and an output 250 which outputs the generated analysis result information. The processor 230 of the biological information processing device generates information for displaying distribution information of a pulse interval found on the basis of the pulse wave information and a reliability index of measurement of the pulse wave information, as the analysis result information, as described above.

The processor 230 of the biological information processing device also generates first analysis result information for identifiably displaying a pulse wave abnormality period in a measurement period, and second analysis result information for displaying at least one of a pulse waveform and a pulse interval waveform during at least a section within the pulse wave abnormality period, as described above.

In the above description, the biological information processing system 200 is implemented by one of the server system, the processing device 400 and the pulse wave measuring device 100. However, this configuration is not limiting. For example, the acquisition of the pulse wave information, the analysis processing on the pulse wave information, the processing of generating the analysis result information, and the processing of outputting the analysis result information may be implemented by distributed processing among a plurality of devices. Specifically, the biological information processing system 200 may be implemented by at least two or more of the server system, processing device 400 and the pulse wave measuring device 100. Alternatively, like the presentation device 300 of FIG. 14, another device may perform a part of the processing by the biological information processing system 200. The biological information processing system 200 according to the embodiment can be implemented by various devices (or combinations of devices).

The technique in the embodiment can also be applied to a method for generating analysis result information (method for producing analysis result information) in which processing of acquiring pulse wave information is performed and then an analysis processing on the pulse wave information is performed, thus generating analysis result information for displaying distribution information of a pulse interval found on the basis of the pulse information and a reliability index of measurement of the pulse wave information.

By using this generation method (production method), it is possible to present whether the displayed distribution information of the pulse interval is reliable or not, in an intelligible form to the viewer (doctor), even in the case of using pulse wave information, which is more easily affected by noises than a electrocardiogram.

The technique in the embodiment can also be applied to a method for generating analysis result information (method for producing analysis result information) in which processing of acquiring pulse wave information is performed and when analysis processing on the pulse wave information is performed, thus generating first analysis result information for identifiably displaying a pulse wave abnormality period for determining an arrhythmia during a measurement period and second analysis result information for displaying at least one of a pulse waveform and a pulse interval waveform during at least a section in the pulse wave abnormality period.

By using this generation method (production method), it is possible to present information to the viewer (doctor) in a form that allows easy understanding of the condition or the like of the user (patient) even when a massive amount of pulse wave information over a long period is acquired.

The biological information processing system 200 and the biological information processing device according to the embodiment include a memory which stores information (for example, programs and various data), and a processor which operates on the basis of the information stored in the memory. The processor performs acquisition processing in which pulse wave information is acquired, processing in which analysis processing on the pulse wave information is performed to generate analysis result information, and output processing in which the analysis result information that is generated is outputted.

The processor then generates information for displaying distribution information of a pulse interval found on the basis of the pulse wave information and a reliability index of measurement of the pulse wave information, as the analysis result information. Alternatively, the processor generates first analysis result information for identifiably displaying a pulse wave abnormality period during a measurement period, and second analysis result information for displaying at least one of a pulse waveform and a pulse interval waveform during at least a section in the pulse wave abnormality period.

The functions of the individual parts of the processor may be implemented, for example, by individual pieces of hardware, or the functions of the individual parts may be implemented by an integrated piece of hardware. For example, the processor may be a CPU (central processing unit). However, the processor is not limited to the CPU, and various kinds of processors such as GPU (graphics processing unit) or DSP (digital signal processor) can be used. The processor may also be a hardware circuit based on ASIC (application specific integrated circuit). The memory may be, for example, a semiconductor memory such as an SRAM or DRAM, a register, magnetic memory such as a hard disk drive, or an optical memory such as an optical disk drive. For example, the memory stores a computer-readable command, and as the processor executes the command, a function of each part of the image processing device. The command in this case may be a command of a command set forming a program, or may be a command which instructs the hardware circuit of the processor to carry out an operation.

The operations in the embodiment are implemented as follows, for example. The processor acquires pulse wave information from the pulse wave sensor 110 and stores the pulse wave information in the memory. The processor then reads out the pulse wave information from the memory, performs analysis processing on the pulse wave information to generate analysis result information, and stores the analysis result information in the memory. In the analysis processing in this case, the processor performs processing of finding a pulse interval and storing the pulse interval in the memory, or processing of finding a reliability index and storing the reliability index in the memory, or the like. Alternatively, the processor performs processing of finding first analysis result information and second analysis result information and storing the first and second analysis result information in the memory. The processor then performs processing of acquiring the analysis result information from the memory and outputting the analysis result information.

Each part of the biological information processing system 200 and the biological information processing device according to the embodiment is implemented as a module of a program which operates on the processor. For example, the acquirer 210 is implemented as an acquisition module which acquired pulse wave information. The processor 230 is implemented as a processing module which performs analysis processing on the pulse wave information to generate analysis result information. The output 250 is implemented as an output module which outputs the generated analysis result information.

While the embodiment has been described in detail above, a person skilled in the art can readily understand that various modifications can be made without substantially departing from the new matters and advantageous effects of the invention. Therefore, all such modifications are understood as included in the scope of the invention. For example, a term described at least once along with a different term having a broader meaning or the same meaning in the specification or drawings can be replaced with the different term in any part of the specification or drawings. Also, the configurations and operations of the biological information processing system and the like are not limited to those described in the embodiment and can be implemented with various modifications.

## Claims

1. A biological information processing system comprising a processor including hardware, wherein
the processor performs:
acquisition processing in which pulse wave information is acquired;
processing in which analysis processing on the pulse wave information is performed to generate analysis result information; and
output processing in which the analysis result information that is generated is outputted, and
the processor generates first analysis result information for identifiably displaying a pulse wave abnormality period during a measurement period, and second analysis result information for displaying at least one of a pulse waveform and a pulse interval waveform during at least a section in the pulse wave abnormality period.

2. The biological information processing system according to claim 1, wherein
the processor generates information for displaying a reliability index of measurement of the pulse wave information during the measurement period, as the first analysis result information.

3. The biological information processing system according to claim 2, wherein
if the reliability index in a first pulse wave abnormality period is higher than the reliability index in a second pulse wave abnormality period,
the processor generates the second analysis result information in such a way that analysis result information corresponding to the first pulse wave abnormality period is displayed preferentially over analysis result information corresponding to the second pulse wave abnormality period.

4. The biological information processing system according to claim 3, wherein
the processor generates the second analysis result information in such a way that analysis result information corresponding to the first pulse wave abnormality period is displayed earlier than analysis result information corresponding to the second pulse wave abnormality period.

5. The biological information processing system according to claim 3, wherein
the processor generates the second analysis result information in such a way that analysis result information corresponding to the second pulse wave abnormality period is displayed with a reduced size, compared with analysis result information corresponding to the first pulse wave abnormality period.

6. The biological information processing system according to claim 2, wherein
the processor further performs section reliability determination processing in which the reliability index corresponding to each section in the measurement period is found on the basis of at least one of body motion information of a user and the pulse wave information.

7. The biological information processing system according to claim 6, wherein
the processor further performs pulse interval calculation processing in which the pulse interval is found on the basis of the pulse wave information, and
the processor performs determination processing on the pulse wave abnormality period on the basis of the pulse interval, and generates the first analysis result information on the basis of a result of the determination processing during each section in the measurement period and the reliability index corresponding to each section.

8. The biological information processing system according to claim 1, wherein
the processor generates information for displaying a least one of body motion information of a user during at least a section in the pulse wave abnormality period and wearing state information of the user, as the second analysis result information.

9. The biological information processing system according to claim 1, wherein
the processor generates information for displaying a display obj ect that indicates the pulse wave abnormality period, as the first analysis result information.

10. The biological information processing system according to claim 1, wherein
the processor generates information for displaying the pulse wave abnormality period and a period that is not the pulse wave abnormality period, in different image display forms from each other, as the first analysis result information.

11. The biological information processing system according to claim 1, wherein
the processor generates the first analysis result information for identifiably displaying the pulse wave abnormality period for determine an abnormal state of a cardiovascular system during the measurement period.

12. The biological information processing system according to claim 11, wherein
the abnormal state of the cardiovascular system is an arrhythmia.

13. The biological information processing system according to claim 12, wherein
the arrhythmia is atrial fibrillation, and
the pulse wave abnormality period is a period which is determined as having an occurrence of the arrhythmia by the analysis processing on the pulse wave information in the processor.

14. A biological information processing device comprising a processor including hardware, wherein
the processor performs:
acquisition processing in which pulse wave information is acquired;
processing in which analysis processing on the pulse wave information is performed to generate analysis result information; and
output processing in which the analysis result information that is generated is outputted, and
the processor generates first analysis result information for identifiably displaying a pulse wave abnormality period during a measurement period, and second analysis result information for displaying at least one of a pulse waveform and a pulse interval waveform during at least a section in the pulse wave abnormality period.

15. A method for generating analysis result information, the method comprising:
performing processing in which pulse wave information is acquired; and
performing analysis processing on the pulse wave information and thus generating first analysis result information for identifiably displaying a pulse wave abnormality period during a measurement period, and second analysis result information for displaying at least one of a pulse waveform and a pulse interval waveform during at least a section in the pulse wave abnormality period.
